(19) Europäisches Patentamt
European Patent Office
Office européen des brevets

(11) EP 1 316 619 A1

(12) EUROPEAN PATENT APPLICATION

(43) Date of publication:
04.06.2003 Bulletin 2003/23

(51) Int Cl.[7]: C12Q 1/68

(21) Application number: 02258072.4

(22) Date of filing: 22.11.2002

(84) Designated Contracting States:
AT BE BG CH CY CZ DE DK EE ES FI FR GB GR IE IT LI LU MC NL PT SE SK TR
Designated Extension States:
AL LT LV MK RO SI

(30) Priority: 30.11.2001 US 337282 P

(71) Applicant: Pfizer Products Inc.
Groton, Connecticut 06340 (US)

(72) Inventors:
- Muehlbauer, Paula A., c/o Pfizer Global Res. & Dev Groton, Connecticut 06340 (US)
- Schuler, Maik J., c/o Pfizer Global Res. & Dev. Groton, Connecticut 06340 (US)

(74) Representative: Hayles, James Richard et al
UK Patent Department,
Pfizer Limited,
Ramsgate Road
Sandwich, Kent CT13 9NJ (GB)

(54) **Method for detecting cells with numerical chromosomal abnormalities**

(57) The invention provides methods for detecting mitotic cells having numerical chromosomal abnormalities and agents inducing numericalchromosomal abnormalities in cells. In a preferred embodiment, the method comprises staining a cell with a mitotic marker: that detects cells in mitosis, and a quantitative DNA stain, indicating the amount of DNA in the mitotic cell. Stained cells are preferably analyzed by flow cytometry.

EP 1 316 619 A1

## Description

### Field of the Invention

This invention relates to the detection of numerical chromosomal abnormalities in cells using a mitotic marker and a quantitative DNA stain. Numerical chromosomal abnormalities such as aneuploidy and polyploidy may be measures of cell toxicity and neoplastic potential.

### Background of the Invention

Numerical chromosomal abnormalities, such as aneuploidy and polyploidy, are associated with numerous diseases, including birth defects, pregnancy wastage and cancer. Exemplary diseases include Down syndrome, Klinefelter's syndrome, Turner's syndrome, Triplo-X syndrome, Tetra-X syndrome, Penta-X syndrome, XYY syndrome, monsomies or polysomies of any chromosome including 7, 10, 11, 13, 18, 21, X, and Y. In addition, numerical chromosomal abnormalities such as polyploidy and aneuploidy are frequently found in spontaneous human abortions.

Exemplary neoplasms that are associated with numerical chromosomal abnormalities include leukemias such as acute lymphoblastic leukemias (ALL), acute non-lymphocytic leukemia (ANL), chronic lymphocytic leukemia (CLL), chronic myelocytic leukemia (CML); solid tumors such as bladder carcinoma, colon cancer, large bowel carcinoma, malignant melanoma and uterine carcinoma. In addition, the role of aneuploidy in the multi-step process of carcinogenesis of rodents has been shown in several tumorigenesis models.

In addition, numerous chemical compounds, such as benomyl, diazepam and griseofulvin have been reported to cause numerical chromosomal abnormalities.

Most existing methods for detecting numerical chromosomal abnormalities rely on chromosome counting methods in which cells in metaphase are stained with a DNA-specific stain like Giemsa and individual chromosomes are counted. This method can be very laborious as it requires manual counting of thousands of cells. Alternatively, the induction of micronuclei is used to detect chemical clastogens. However this method does not easily discriminate between clastogens and aneugens and is not sensitive to chromosomal non-disjunction. Yet other techniques include fluorescence in situ hybridization (FISH) techniques using chromosome-specific DNA probes which can anneal to complementary sequences on chromosomes and thus act as specific markers. These DNA probes include region-specific or whole chromosome probes that result in uniform or almost uniform series of fluorescent signals throughout the length of a particular chromosome.

However, all of these techniques can be laborious and time-consuming. Accordingly, there is a need for a fast, inexpensive and reliable method for identifying numerical chromosomal abnormalities. Such a method could be used for detecting numerical chromosomal abnormalities in biological samples and for identifying compounds that induce numerical chromosomal abnormalities.

The contents of all cited references including literature references, issued patents, published patent applications as cited throughout this application are hereby expressly incorporated by reference in their entirety.

### Summary of the Invention

The invention provides methods for detecting one or more numerical chromosomal abnormalities in a cell. The chromosomal abnormality can be an abnormal ploidy, such as polyploidy or aneuploidy. In a preferred embodiment, the method comprises marking a cell with a mitotic marker and a quantitative DNA stain, to thereby quantify the amount of DNA in the cell at mitosis, and detect the presence of one or more numerical chromosomal abnormalities in the mitotic cell. The mitotic marker can be an agent that detects a characteristic of a mitotic cell, e.g., phosphorylation of a histone that correlates with chromatin condensation at mitosis, e.g., the phopsphorylation of histone H3 on Serine-10. The agent can be an antibody, such as the 6G3 monoclonal antibody. Any quantitative DNA stain can be selected, e.g., from the group consisting of intercalating dyes, minor groove binders or a cyanine dye. A preferred quantitative DNA stain is propidium iodide. The method preferably includes subjecting the cell stained with a mitotic marker and a quantitative DNA stain to flow cytometry for analysis of the stained cells.

The invention also provides methods for detecting one or more numerical chromosomal abnormalities in one or more cells in a population of cells, comprising staining the population of cells with a mitotic marker and a quantitative DNA stain marker, to thereby quantify the amount of DNA in the cells at mitosis, and determine the presence of one or more numerical chromosomal abnormalities in one or more cells.

The invention also provides methods for isolating one or more cells having one or more numerical chromosomal abnormalities in a population of cells, comprising staining the population of cells with a mitotic marker and a quantitative DNA stain, subjecting the cells to flow cytometry, thereby visualizing the DNA content of the cells, to thereby identify cells having one or more chromosomal abnormalities, and separating the one or more cells having one or more chromosomal abnormalities from the population of cells. A preferred method of analysis and separation includes flow cytometry. The method may comprise separating at least two different subpopulations of cells from each other and from the population of cells. The methods of the invention can be applied to any type of cell undergoing mitosis or cells that can be stimulated by a mitogen to undergo mitosis, e.g., an eukaryotic cell. Exemplary cells include mammalian cells, such as human cells. Preferred cells include somatic and germ cells. The cells can be from a biological sample, e.g., a biopsy, or from cultured cells, e.g., cells from a cell line.

The invention further provides assays for identifying compounds that induce a numerical chromosomal abnormality in a cell. In a preferred embodiment, an assay comprises contacting a population of cells with a test compound for a time sufficient to induce any potential numerical chromosomal abnormality in daughter cells, subjecting the cells to a method of the invention, and determining the presence of the chromosomal abnormality in cells, such that a higher number of cells having a chromosomal abnormality in the population of cells contacted with the test compound relative to a similar population of cells that was not contacted with the test compound, indicates that the test compound induces a numerical chromosomal abnormality in cells. Any eukaryotic cell, e.g., mammalian, e.g., human cell can be used is this assay.

In another embodiment, the invention provides methods for determining the presence of cells containing one or more chromosomal abnormalities in a population of cells from a human subject or animal. The method preferably comprises obtaining a sample of cells from a human subject or animal, subjecting the sample of cells to a method of the invention, to thereby determine the presence of cells containing one or more numerical chromosomal abnormalities in a human subject or animal. Exemplary diseases include various types of cancer, germ cell aneusomies as well as genetic diseases, such as trisomies and monosomies.

The invention further provides kits for detecting numerical chromosomal abnormalities in cells. The kit may be a kit for identifying compounds that are cytotoxic or for identifying numerical chromosomal abnormalities in samples. A kit may comprise one marker, such as a mitotic marker or a quantitative DNA stain. In a preferred embodiment, a kit comprises both a mitotic marker and a quantitative DNA stain. Other components that a kit may comprise include additional markers, detection reagents, buffers, negative or positive controls, and devices for obtaining a biological sample. The kit may also comprise instructions for use.

The invention also provides compositions comprising a mitotic marker and a quantitative DNA stain.

The invention provides at least the advantage of providing a dramatically increased speed of analysis of numerical chromosomal abnormalities in cells, reduced variability in data, increased statistical power and overall improved assessment of numerical aberrations in mitotic populations.

**Brief Description of the Drawings**

Fig. 1A represents a 2 parameter dot plot (FL2-W/FL2-A) obtained by flow cytometric analysis of human lymphocytes, showing the appropriate "gating set-up" for assuring complete acquisition of the cycling population of cells.

Fig. 1B represents a 2 parameter dot plot (anti-H3 mAb/FL2-A) obtained by flow cytometric analysis of normal human lymphocytes, showing the appropriate "gating set-up" for identifying the different populations of mitotic cells based on DNA content (i.e. G0-G1-S and G2) for multiple cell cycles.

Fig. 2A represents an example of a 2 parameter dot plot (FL2-W/FL2-A) obtained by flow cytometric analysis of human lymphocytes treated with Noscapine, showing the "gated" cycling population of cells with increasing DNA content.

Fig. 2B represents an example of a 2 parameter dot plot (anti-H3 mAb/FL2-A) obtained by flow cytometric analysis of human lymphocytes treated with Noscapine, showing the appropriate "gating set-up" for quantifying the hypo-diploid (R2), diploid (R3), hyper-diploid (R4) and polyploid (R5) cells.

Fig. 3A represents a DNA "set-up" histogram example from MODFIT 3.0 software, showing the manual alignment of the G1 and G2 peaks of DNA.

Fig. 3B represents a DNA "analysis" histogram example from MODFIT 3.0 software, showing the calculated number of cells in G1, G2 and S phases, cell aggregates and cell debris.

Fig. 4 is a graph showing the comparison of manual and flow cytometric analysis of polyploid cells in a population of human lymphocytes treated with different amounts of Noscapine.

Fig. 5 is a graph showing the percentage of hypo-diploid, hyper-diploid and polyploid cells in a population of human lymphocytes treated with different amounts of potassium cyanide (KCN), as determined by flow cytometric analysis.

Fig. 6 is a graph showing the percentage of hypo-diploid, hyper-diploid and polyploid cells in a population of human lymphocytes treated with different amounts of cytochalasin B, as determined by flow cytometric analysis.

Fig. 7 is a graph showing the percentage of hypo-diploid, hyper-diploid and polyploid cells in a population of human lymphocytes treated with different amounts of colcemid, as determined by flow cytometric analysis.

Fig. 8 is a graph showing the percentage of hypo-diploid, hyper-diploid and polyploid cells in a population of human lymphocytes treated with different amounts of griseofulvin, as determined by flow cytometric analysis.

Fig. 9 is a graph showing the percentage of hypo-diploid, hyper-diploid and polyploid cells in a population of human lymphocytes treated with different amounts of noscapine, as determined by flow cytometric analysis.

**Detailed Description of the Invention**

The invention is based at least in part on the discovery that labeling a population of cycling cells with of a marker of mitosis and staining for DNA content and visualization of the stained cells via flow cytometry permits a straightforward methods of detecting cells with numerical chromosomal abnormalities in population of cells. The results show the numbers of mitotic cells having extra or missing chromosomes or a multiple of extra chromosomes.

In the detection methods, identification methods, isolation methods and assay aspects of this invention, cells may be treated first with a mitotic marker and then with a quantitative DNA stain or the order may be reversed. Alternatively, cells may be treated first with a quantitative DNA stain followed by DNA quantification. Those cells showing numerical chromosomal abnormalities could then be identified as mitotic or non-mitotic by treatment with a mitotic marker. As another alternative, cells may first be identified as mitotic or non-mitotic and then treated with a quantitative DNA stain to identify numerical chromosomal abnormalities. All of the foregoing alternatives are within the bounds of the invention.

The terms used herein have their usual meaning in the art, however, to even further clarify the present invention, for convenience, the meaning of certain terms and phrases employed in the specification, examples, and appended claims are provided below.

An "aneugen" is an agent, such as a chemical, causing aneuploidy. A "non-aneugen" is an agent that does not cause aneuploidy.

"Aneuploidy" and "polyploidy" are conditions in which a cell has a number of chromosomes different from the usual haploid number ("n") or diploid number ("2n"). In humans, the normal haploid number of chromosomes is 23, and the diploid number is 46. A normal cell should have a diploid chromosome content of 46 chromosomes. An aneuploid cell has a number of chromosomes that is not equal to a whole multiple of the normal haploid number. For example, an aneuploid cell can be a cell having a trisomy, i.e., a cell having three copies of one chromosome, or a monosomy, i.e., a cell having a single copy of one chromosome. Aneuploidy can result from chromosomal non-disjunction during mitosis. A polyploid cell is a cell having a number of chromosomes that is some multiple of the normal haploid number greater than the usual diploid number, i.e., 2n. For example, a polyploid cell can be triploid (n=69) or tetraploid (n=92) cell.

The term "biological sample" refers to a sample obtained from an organism or from components (e.g., cells) of an organism. The sample may be of any biological tissue or fluid. Frequently the sample will be a "clinical sample" which is a sample derived from a patient. Such samples include, but are not limited to, sputum, blood, blood cells (e. g., white cells), tissue or fine needle biopsy samples, urine, peritoneal fluid, and pleural fluid, or cells therefrom. Biological samples may also include sections of tissues such as frozen sections taken for histological purposes.

"Numerical chromosomal abnormality" in a cell refers to the presence in the cell of more additional chromosomes or portions thereof or the lack of more chromosomes or portions thereof, relative to a normal cell.

"Chromosomal content" of a cell refers to the number of chromosomes and portions thereof. A normal chromosomal content for a diploid cell is 2n and for a haploid cell n. The normal chromosomal content of a diploid cell in mitosis is 4n.

"Quantitative DNA stain" refers to an agent that permits the detection and quantification of the DNA of a cell. A preferred quantitative DNA stain is an agent that stains DNA, e.g., an intercalator. An exemplary quantitative DNA stain is propidium iodide.

A "hypo-diploid" cell is a cell having less chromosomal DNA than a normal diploid cell of the same species.

A "hyper-diploid" cell is a cell having more chromosomal DNA than a normal diploid cell of the same species.

"Label" and "detectable label" refer to a molecule capable of detection, including, but not limited to, radioactive isotopes, fluorophores, chemiluminescent moieties, enzymes, enzyme substrates, enzyme cofactors, enzyme inhibitors, dyes, metal ions, ligands (e.g., biotin or haptens) and the like. The term "fluorescer" refers to a substance or a portion thereof which is capable of exhibiting fluorescence in the detectable range. Examples of labels that may be used under the invention include fluorescein, rhodamine, dansyl, umbelliferone, Texas red, luminol, NADPH, alpha - beta -galactosidase and horseradish peroxidase.

A "marker" can be an agent that marks some characteristic of a cell, e.g., the stage of the cell cycle or the presence and/or amount of a cell component, e.g., DNA.

"Mitotic marker" is an agent that marks cells, which are in mitosis,. A preferred mitotic marker is an agent that detects phosphorylation of histone H3 on the Ser-10site.

"Ploidy" refers to the degree of repetition of the normal number of chromosomes. A cell having an "ploidy" is a cell having a number of chromosomes different from that of a normal cell of the same species and can be, e.g., an aneuploid or polyploid cell. "Measurement of ploidy" of a cell refers to the determination of the number of chromosomes in the cell.

"Small molecule" as used herein, is meant to refer to a composition, which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays of the invention to identify compounds causing numerical chromosomal abnormalities.

"Stain" refers to any agent that can detect a target e.g., a DNA dye.

In one embodiment, the invention provides a method for determining the chromosomal content, e.g., ploidy, of a cell, comprising labeling the cell with a mitotic marker and staining for DNA content, to thereby quantify the amount of DNA of the cell at mitosis, and thereby determine the chromosomal content of the cell. The presence of labeling obtained with the mitotic marker indicates that the cell is undergoing mitosis. The amount of staining obtained with the quantitative DNA stain correlates with the amount of DNA in the cell, e.g., the number of chromosomes or portions thereof in the cell. The use of the mitotic marker allows to limit the analysis of DNA content to mitotic cells. Determining the chromosomal content of a cell includes determining the presence of aneuploidy or polyploidy or the presence or absence of a portion of chromosomes in the cell. Accordingly, a cell that is mitotic and has a DNA content other than 4n is judged to have a numerical chromosomal abnormality. If the DNA content is some multiple of the normal haploid number greater than 4n, such as 8n, then the cell is deemed polyploid. If the content is not substantially equal to 4n and not a whole multiple of n greater than 4n, then the cell is deemed aneuploid. A variety of techniques can be used to detect and/or quantify the stains. A preferred method is flow cytometry.

The presence of numerical chromosomal abnormalities in a cell, e.g., aneuploidy and polyploidy, might also be assessed simply by measuring the DNA content of cells without regard to mitotic state. However, by preferentially evaluating cells in mitosis, detection of numerical chromosomal abnormalities is enhanced. This occurs because mitotic cells are selected from the cycling population of cells, largely eliminating several sources of background error, including dead cells, cells undergoing programmed cell death (also known as apoptosis), and cell fragments or debris, all of which could appear by DNA content measures to be numerical chromosomal abnormalities.

The cell can be a cell within a population of cells. Accordingly, the invention provides methods for determining the presence of numerical chromosomal abnormalities of one or more cells in a population of cells, comprising staining the population of cells with a mitotic marker and quantitative DNA stain, to thereby quantify the amount of DNA of the cells at mitosis. The invention provides methods for quantifying the number of cells having one or more numerical chromosomal abnormalities in a population of cells. For example, the method may comprise counting the mitotic cells having a chromosomal number different from 4n.

The methods of the invention are applicable to cell lines as well as to cultures of primary cells and biological samples, e.g., biopsies, from animals. Any cell that undergoes mitosis can be used. Preferred cells include eukaryotic cells. The cell can be a vertebrate cell, e.g., a mammalian cell, e.g., a human, a bovine, ovine, porcine, equine, canine, feline, non-human primate, mouse, or rat cell. The cell can be from any organ or tissue of an eukaryote, e.g., a blood cell, an epithelial cell, a bone marrow cell, a lung cell, a kidney cell or an amniotic fluid cell. The methods of the invention can also be applied to yeast cells, *Drosophila* cells, *Xenopus* cells, *C. elegans* cells, or any other cell having chromosomes.

Cell lines for use, e.g., in identifying agents that cause numerical chromosomal abnormalities, can be obtained, e.g., from the American Type Culture Collection (ATCC) 10801 University Blvd., Manassas, VA 20110-2209.

Biological samples of cells may be obtained from an individual using either "invasive" or "non-invasive" sampling means. A sampling means is said to be "invasive" if it involves the collection of cells from within the skin or organs of an animal. Examples of invasive methods include blood collection, semen collection, needle biopsy, pleural aspiration, umbilical cord biopsy, amniotic fluid biopsy, etc. Examples of such methods are discussed by Kim, C. H. et al. (J. Virol. 66:3879-3882 (1992)); Biswas, B. et al. (Annals NY Acad. Sci. 590:582-583 (1990)); Biswas, B. et al. (J. Clin. Microbiol. 29:2228-2233 (1991)).

In contrast, a "non-invasive" sampling means is one in which the cells are recovered from an internal or external surface of the animal. Examples of such "non-invasive" sampling means include "swabbing," collection of tears, saliva, urine, fecal material, sweat or perspiration, hair etc. Such collection may be accomplished by swabbing nasal, oral, rectal, vaginal or aural orifices, by contacting the skin or tear ducts, by collecting hair follicles, etc. As used herein, "swabbing" denotes contacting an applicator/collector ("swab") containing or comprising an adsorbent material to a surface in a manner sufficient to collect cells.

The population of cells can be a pure population of cells, e.g., cells from a cell line, or a diverse population of cells. In one embodiment, the cells are peripheral blood mononuclear cells (PBMCs). A population consisting of different types of cells can further be enriched in one or more subtypes. For example, lymphocytes can be isolated from PBMCs. Isolation of subpopulations of cells can be performed by various methods, such as affinity purification or panning methods using an antibody reacting specifically with a cell surface marker.

The methods of the invention can be used on a single cell. The methods of the invention are preferably used on a population of at least 10, preferably at least $10^2$, $10^3$, $10^4$, $10^5$, $10^6$, $10^7$ or $10^8$ cells.

In one embodiment, the cells are incubated with a spindle block (e.g., Colcemid® or Colchicine (GIBCO, BLR) and TN16 (Wako Pure chemical)). For example, cells can be exposed to Colcemid (about 0.1 µg/ml; GIBCO) for 1-3 hours prior to being fixed. A "spindle block" is an agent that causes cells to accumulate at mitosis.

Before or after staining, the cells are optionally treated with solvents to fix, and optionally permeabilize, the cell membranes. A number of fixatives and fixation conditions are suitable for this purpose. Useful fixatives include, but are not limited to, formaldehyde, e.g., 2% paraformaldehyde; cold methanol; cold ethanol, e.g., 70% ethanol) . Cell fixation can also be accomplished by incubation in a 3.7% solution of paraformaldehyde for about 15-30 minutes.

Prior to fixation, the cells can be pipetted through a small bore pipette to ensure single cell suspension. The cells can also be filtered, e.g., through a 35 µm strainer cap (Becton Dickinson; Franklin Lakes, N.J.) for this purpose.

Generally, cells are fixed in a particular reagent and for a time sufficient for the particular method used, e.g., flow cytometry. A suitable fixation method for a particular cell type can be determined according to methods known in the art, e.g., by conducting assays with different fixation methods to determine which fixation method allows suitable staining of the cells for using in the particular method.

Depending on the mitotic marker and/or quantitative DNA stain used, it may be necessary to permeabilize a cell prior to labeling with the mitotic marker. For example, prior to antibody labeling of cells, the cells are preferably permeabilized. Permeabilization is utilized to allow bulky markers to enter the cellular space that would ordinarily be impermeant with respect to the cellular membrane. Permeabilization can be accomplished by incubating the cells in reagents, such as acetone, ethanol, DMSO or various detergents, e.g., Tween-80 or Triton X-100. For example, the cells can be permeabilized by incubation in about 0.05-1.0% Triton X-100 during or after fixing, if any, of the cells.

Generally, when cells are permeabilized, they are permeabilized with a reagent and for at time sufficient for the particular stain, marker and/or detection agent used to reach their target on or in the cells. A suitable permeabilization method for the particular cells and reagents used in the method can be determined according to methods known in the art, e.g., by conducting assays with several different permeabilization methods, such as to determine which permeabilization method allows suitable staining of the cells.

A large variety of fixatives, fixation conditions, and permeabilization agents are known in the art, and other methods of fixing or permeabilizing sample cells in conjunction with the stains of the present invention will be obvious to one of ordinary technical skills.

A preferred quantitative DNA stain is propidium iodide (PI; Molecular Probes, product no. P-3566; Calbiochem; La Jolla, Calif.). Other useful quantitative DNA stains are fluorescent nucleic acid stains. A variety of appropriate nucleic acid stains are known in the art, including but not limited to, thiazole orange, ethidium homodimer, ethidium bromide, HOECHST 33258, and DAPI. Nucleic acid stains can be intercalating dyes, such as phenanthridines and acridines, e.g., ethidium bromide and propidium iodide; minor-groove binders, such as DAPI and the Hoechst dyes; or not belonging in either of these two categories, such as acridine orange, 7-AAD and hydroxystilbamidine. Cyanine dyes are dyes of choice based on their high molar absorptivity, very low intrinsic fluorescence, large fluorescence enhancements upon binding to nucleic acids and moderate to very high affinity for nucleic acids, with little or no staining of other biopolymers. Some nucleic acid stains are cell-impermeant and other stains are cell permeant stains. Cell permeant stains do not require permeabilization of cells prior to staining. Exemplary cell permeant dyes are the cyanine dyes (SYTO nucleic acid stains); hexidium iodide, dihydroethidium, and ethidium homodimers.

Other dyes available, include the intercalators 7-amino actinomycin D and actinomycin D, multicolor hydroxystilbamidine, Long-Wavelength LDS751 and Neurtrace Fluorescent Nissl Stains. Another quantitative DNA stain is the base analog 5-bromo-2'-deoxyuridine (BrdU, available from Sigma Chemical Co.). Additional useful nucleic acid stains are described in the international applications WO 93/06482 (published Apr. 1, 1993); WO 94/24213 (published Oct.

27, 1994); U.S. Pat. No. 5,321,130 to Yue et al., 1994; U.S. Pat. No. 5,410,030 to Yue et al., 1995; U.S. Pat. No. 5,436,134 to Haugland et al., 1995; and U.S. Pat. No. 5,437,980 to Haugland et al., 1995. The use of an appropriate nucleic acid stain in conjunction with a mitotic marker of the present invention can be selected to allow simultaneous or sequential observation of mitotic cells and DNA content.

Propidium iodide (PI; Calbiochem; La Jolla, Calif.) can be added to a cell population at a concentration of at least about 0.1 μg/ml to about 100 μg/ml, preferably at least about 0.1 to about 10 μg/ml, and most preferably from about 1 to about 5 μg/ml. The cells can be incubated for about 1 minute to about 2 hours, preferably for about 10 minutes to one hour, and most preferably for about 15 to about 30 minutes at room temperature, optionally in the darkness. Incubation can also be carried out on ice, for at least about 1 minute, preferably at least about 5 minutes, and most preferably for at least about 15 minutes.

Generally, cells are stained with a quantitative DNA stain in an amount and for a time sufficient to permit detection and quantification of the DNA in the cells. Suitable staining procedures for a particular cell type and detection method can be determined according to methods known in the art, e.g., by conduction assays using several DNA stains, concentration and incubation times, such as to determine which conditions permit suitable staining of the cells.

Staining of cells for DNA content may be accompanied by treatment of the cells with an agent that prevents staining of nucleic acids other than chromosomal DNA, e.g., RNA. In an illustrative embodiment, the cells are treated with an agent that degrades RNA, e.g., an RNase. For example, RNase A (BD Clontech; Palo Alto, California) can be added at about 100-500 μg/ml.

Generally, cells are incubated with an agent that prevents staining of nucleic acids other than chromosomal DNA in a concentration and for a time sufficient to permit predominant staining of the chromosomal DNA over staining of the other nucleic acids. Suitable conditions can be determined according to methods in the art, e.g., by conducting assays using different agents in different conditions, such as to determine suitable conditions for the method used.

A preferred mitotic marker is an agent that specifically detects mitotic cells by detecting a characteristic of mitotic cells (M) that is essentially absent or undetectable in non-mitotic cells. In a preferred embodiment, a mitotic marker is a marker that detects chromatin condensation, which occurs at mitosis, but not during interphase (i.e., not in G0, G1, S or G2). An even more preferred mitotic marker is an agent that detects phosphorylation of histone H3 on Ser-10 (anti-H3-P antibody) (Juan et al. (1998) Cytometry 32:71). The agent can be an antibody, such as a monoclonal antibody. A preferred antibody is the anti-H3-P antibody 6G3, which is commercially available from Cell Signaling Technology (Berverly, MA) (Juan et al., *supra*). The antibody detects specifically the phosphorylated form of the Ser-10 residue, and not the unphosphorylated form. Mitotic cells from prophase to telophase are detected by the anti-H3-P antibody. On the contrary, the binding of the antibody to chromatin of interphase cells (i.e., Go, $G_1$, S and $G_2$) is several times weaker. Therefore, the only cells that bind this antibody are cells in mitosis.

Other mitotic markers that can be used include the TG-3 monoclonal antibody (Anderson 1998, Experimental Cell Res 238, 498-502).

Generally, cells are labeled with a mitotic marker at a concentration and for a time sufficient to permit detection of mitotic cells in the particular method used, e.g., flow cytometry. Suitable conditions can be determined according to methods known in the art, e.g., incubation of cells in different conditions with a mitotic marker, to identify suitable conditions.

In certain embodiments, cells can be stained with one or more additional markers. For example, a third marker may be another mitotic marker or DNA marker, to confirm the results obtained with the other two markers. Alternatively, another marker could be a marker that detects the size or the granularity of the cells or cellular organelles.

For example, an additional marker can be any probe or stain that selectively stains a cellular organelle such as the cell membrane, nucleus, Golgi apparatus, endoplasmic reticulum, lysosomes, or is a second mitochondrial stain, such as rhodamine 123, or the fixable mitochondrial stains described in U.S. Pat. No. 5,459,268 to Haugland et al. (1995). Specific examples of additional markers include stains for acidic organelles, such as LYSOTRACKER (Molecular Probes, Eugene, Oreg.) and other stains for lysosomes described in U.S. patent no. 6,291,203. The use of stains that are selective for lysosomes in combination with the stains of the present invention allows multiple color visualization of mitotic cells, DNA content, and, e.g., the mitochondria and acidic organelles in cells.

The mitotic marker and/or the quantitative DNA stain can be agents which are not themselves detectable, but which can be detected by a secondary reagent or "detection reagent." For example, a marker can be an antibody. The antibody can be directly labeled, such as with a label set forth below. Alternatively, the antibody can be reacted with a secondary (or second) reagent recognizing the antibody. Various secondary reagents are known in the art, e.g., FITC. The antibody can also be linked to biotin and the secondary reagent can be linked to avidin or streptavidin. Generally, the secondary reagent will carry the label. The reagent carrying the label is referred to as "detection reagent."

Generally, the detection reagent incorporates a means for producing a detectable response. A detectable response means a change in, or occurrence of, a parameter in a test system which is capable of being perceived, either by direct observation or instrumentally, and which is a function of the presence of a specifically targeted member of a specific binding pair in a cell sample. Such detectable responses include the change in, or appearance of, color, fluorescence, reflectance, pH, chemiluminescence, or infrared emission. Appropriate labels to provide a detectable response include, but are not limited to, a visible or fluorescent dye, an enzyme substrate that produces a visible or fluorescent precipitate upon enzyme action (for example, the action of horseradish peroxidase upon diaminobenzidine), an enzyme substrate that produces fluorescence or visible color upon enzymatic conversion, visible or fluorescent labeled latex microparticles, or a signal that is released by the action of light upon the reagent (e.g. a caged fluorophore that is activated by photolysis, or the action of light upon diaminobenzidine).

Fluorescent moieties or labels of interest include coumarin and its derivatives, e.g. 7-amino-4-methylcoumarin, aminocoumarin, bodipy dyes, such as Bodipy FL, cascade blue, fluorescein and its derivatives, e.g. fluorescein isothiocyanate, Oregon green, rhodamine dyes, e.g. Texas red, tetramethylrhodamine, eosins and erythrosins, cyanine dyes, e.g. Cy2, Cy3, Cy3.5, Cy5, Cy5.5, Cy7, FluorX, macrocyclic chelates of lanthanide ions, e.g. quantum dye™, fluorescent energy transfer dyes, such as thiazole orange-ethidium heterodimer, TOTAB, dansyl, etc. Individual fluorescent compounds which have functionalities for linking to a detection reagent, or which can be modified to incorporate such functionalities include, e.g., dansyl chloride; fluoresceins such as 3,6-dihydroxy-9-phenylxanthydrol; rhodamineisothiocyanate; N-phenyl 1-amino-8-sulfonatonaphthalene; N-phenyl 2-amino-6-sulfonatonaphthalene; 4-acetamido-4-isothiocyanato-stilbene-2,2'-disulfonic acid; pyrene-3-sulfonic acid; 2-toluidinonaphthalene-6-sulfonate; N-phenyl-N-methyl-2-aminoaphthalene-6-sulfonate; ethidium bromide; stebrine; auromine-0,2-(9'-anthroyl)palmitate; dansyl phosphatidylethanolamine; N,N'-dioctadecyl oxacarbocyanine: N,N'-dihexyl oxacarbocyanine; merocyanine, 4-(3'-pyrenyl) stearate; d-3-aminodesoxy-equilenin; 12-(9'-anthroyl)stearate; 2-methylanthracene; 9-vinylanthracene; 2,2'(vinylene-p-phenylene)bisbenzoxazole; p-bis(2-methyl-5-phenyl-oxazolyl))benzene; 6-dimethylamino-1,2-benzophenazin; retinol; bis(3'-aminopyridinium) 1,10-decandiyl diiodide; sulfonaphthylhydrazone of hellibrienin; chlorotetracycline; N-(7-dimethylamino-4-methyl-2-oxo-3-chromenyl)maleimide; N-(p-(2benzimidazolyl)-phenyl)maleimide; N-(4-fluoranthyl)maleimide; bis(homovanillic acid); resazarin; 4-chloro-7-nitro-2,1,3-benzooxadiazole; merocyanine 540; resorufin; rose bengal; and 2,4-diphenyl-3(2H)-furanone. (*see*, *e.g.*, Kricka, 1992, Non-isotopic DNA Probe Techniques, Academic Press San Diego, Calif.). Many fluorescent tags are commercially available from SIGMA chemical company (Saint Louis, Mo.), Amersham, Molecular Probes, R&D systems (Minneapolis, Minn.), Pharmacia LKB Biotechnology (Piscataway, N.J.), CLONTECH Laboratories, Inc. (Palo Alto, Calif.), Chem Genes Corp., Aldrich Chemical Company (Milwaukee, Wis.), Glen Research, Inc., GIBCO BRL Life Technologies, Inc. (Gaithersberg, Md.), Fluka Chemica-'Biochemika Analytika (Fluka Chemie AG, Buchs, Switzerland), and Applied Biosystems (Foster City, Calif.) as well as other commercial sources known to one of skill in the art.

Chemiluminescent labels include luciferin and 2,3-dihydrophthalazinediones, e.g., luminol. Isotopic moieties or labels of interest include $^{32}$P, $^{33}$P, $^{35}$S, $^{125}$I, $^{2}$H, $^{14}$C, and the like (*see* Zhao et al. (1995) Gene 156:207; Pietu et al. (1996) Genome Res. 6:492).

Labels may also be members of a signal producing system that act in concert with one or more additional members of the same system to provide a detectable signal. Illustrative of such labels are members of a specific binding pair, such as ligands, e.g. biotin, fluorescein, digoxigenin, antigen, polyvalent cations, chelator groups and the like, where the members specifically bind to additional members of the signal producing system, where the additional members provide a detectable signal either directly or indirectly, e.g., antibody conjugated to a fluorescent moiety or an enzymatic moiety capable of converting a substrate to a chromogenic product, e.g. alkaline phosphatase conjugate antibody and the like. Exemplary couples of agents that can be used include an enzyme and an enzyme substrate; an antigen and an antibody, biotin and avidin (or streptavidin), immunoglobulin and protein A or protein G, and carbohydrate and lectin.

Enzyme substrates as a means to produce a fluorescent precipitate in the presence of the appropriate enzyme are further described in U.S. Pat. No. 5,316,906 to Haugland et al. (1994) and U.S. Pat. No. 5,443,986 to Haugland et al. (1995).

Exemplary labeled antibodies that recognize the constant regions of antibodies, which can be used, e.g., with the 6G3 antibody, include antibodies labeled with a fluorochrome, e.g., Alexa Fluor 488 (Molecular Probes, A-11011), fluorescein isothiocyanate (FITC), phycoerythrin (PE), R-phycoerythrin (R-PE), Cy-Chrome™, and PerCP (BD PharMingen). Antibodies can also be conjugated to allophycocyanin (APC) and Texas Red™. Such labeled secondary antibodies are well known in the art and available from numerous commercial sources.

Other appropriate additional detection reagents include selected fluorescent pH or metal ion indicators such as those described in U.S. Pat. No. 5,453,517 to Kuhn et al. (1995); U.S. Pat. No. 5,405,975 to Kuhn et al. (1995); and in Haugland, supra, 1992 HANDBOOK, Sets 20-22. Yet other labels of interest include those described in U.S. Pat. No. 5,563,037; WO 97/17471 and WO 97/17076.

Examples of distinguishable labels for use on different markers that will be measured together are well known in the art and include: two or more different emission wavelength fluorescent dyes, like Cy3 and Cy5, combination of fluorescent proteins and dyes, like phicoerythrin and Cy5, two or more isotopes with different energy of emission, like $^{32}$P and $^{33}$P, gold or silver particles with different scattering spectra, labels which generate signals under different treatment conditions, like temperature, pH, treatment by additional chemical agents, etc., or generate signals at different time points after treatment. Using one or more enzymes for signal generation allows for the use of an even greater variety of distinguishable labels, based on different substrate specificity of enzymes (alkaline phosphatase/peroxidase).

A preferred method of the invention comprises fixing cells in a solution and for time adequate for fixing the cells. The cells are then incubated in a solution comprising a reagent for permeabilization of the cells that is adequate for the stain used in the method. The cells are preferably incubated in a solution containing a detergent for a time sufficient to permeabilize the cells for a particular mitotic marker and optionally for secondary detection reagents. A permeabilization step is optional and is only necessary when a non-cell permeant marker or detection agent is used. The cells can then be incubated in the presence of a first marker, e.g., a mitotic marker, in an amount and for a time sufficient for the marker to bind to at least 70%, preferably at least about 80% and most preferably at least about 100% of its target molecule. After incubation with the first marker, the cells can be incubated with a secondary detection agent in an amount and for a time sufficient to react with at least about 70%, preferably at least about 80% and most preferably at least about 100% of its target molecule. The cells can be incubated with a second marker, such as a quantitative DNA stain, at a concentration and for a time sufficient to react with at least about 70%, preferably at least about 80% and most preferably at least about 100% of its target molecule.

The order in which the markers are added to the cells is critical. For example, the quantitative DNA stain may not be added to the cell before the mitotic marker. A secondary detection agent is added after having stained the cell with the agent that the secondary detection agent recognizes. A secondary detection agent can be added to cells after the cells have been stained with one or two or more markers.

Accordingly, in an exemplary embodiment, about 1-10 x $10^6$ cells are washed in PBS and fixed in 70% ethanol at -20 °C. The cells are then centrifuged, and permeabilized by incubation in about 0.05 to about 1% PBS/Tween 80, preferably about 0.05 to 0.5%, and most preferably in about 0.1 to 0.2% PBS/Tween 80 for a time sufficient to permeabilize the cells. The adequate time will depend on the type of cell, and can be determined in small-scale assays. Generally, an incubation from about 5 minutes to about 30 minutes, preferably from about 10 minutes to about 20 minutes and most preferably from about 10 minutes to about 15 minutes at room temperature will be appropriate for permeabilizing the cells. The cells are centrifuged, and the cell pellet resuspended in a solution containing H3 (ser 10) 6G3 antibody diluted about 100 to 1000 fold, preferably about 300 to 700 fold, and even most preferably from about 400 to 500 fold from the solution commercially available from Cell Signaling Technology (Berverly, MA). The cells are incubated for about 10 minutes to one hour, preferably from about 30 minutes to one hour, and most preferably for about 40 minutes, at 37 °C. PBS is then added to the solution, mixed with the cells, and the cells are centrifuged. The cell pellet is then resuspended in Alexa Fluor 488 labeled secondary antibody and incubated for about 10 minutes to 60 minutes, preferably from about 20 minutes to about 40 minutes and most preferably for about 30 minutes at 37 °C. PBS is added to the cells, mixed with the cells, the cells centrifuged and the pellet resuspended in a solution comprising propidium iodide and, optionally RNase. The propidium iodide is preferably present at a concentration of at least about 0.1 μg/ml to about 100 μg/ml, preferably at least about 0.1 to about 10 μg/ml, and most preferably from about 1 to about 5 μg/ml. RNase is preferably added at a concentration of about 10 μg/ml to about 1000 μg/ml, more preferably from about 50 μg/ml to about 500 μg/ml, and most preferably from about 100 μg/ml to about 300 μg/ml. Incubation is preferably performed on ice for at least about one minute, preferably for at least about 10 minutes. The cells can be stored at 4 °C prior to FACS analysis. The cells can be analyzed for DNA content (fluorescent intensity), aneuploidy (including polyploidy) and cell cycle analysis by flow cytometry.

A preferred method for analyzing the cells staining with one or both markers is by flow cytometry or laser scanning cytometry, using, multi- parameter (or color) displays. In an even more preferred embodiment, cells that are stained with a mitotic marker and a quantitative DNA stain are subjected to two-parameter flow cytometry. This allows the visualization of the mitotic cells in reference to DNA content , and allows e.g., the visualization of different subpopulations of mitotic cells having an abnormal chromosomal content, e.g., aneuploidy or polyploidy. In addition, flow cytometry permits the isolation of subpopulations of mitotic cells, e.g., all the cells that have fewer chromosomes can be isolated in one tube and the all the cells having extra chromosomes can be isolated in a second tube. Accordingly, multiple subpopulations of cells can be separated out from each other and from the other cells. Such populations of cells can then further be examined.

Flow cytometry can be performed with a fluorescent activated cell sorter (FACS) as further described herein and as known in the art. Exemplary FACS machines that can be used include FACS-Calibur (Becton Dickinson; Mountain View, Calif.) and a Coulter flow cytometer (Hialeah, Fla., USA) EPICS Elite$^R$. Quantification can be performed using CellQuest (Becton Dickinson; Mountain View, Calif.), WinList (Verity Software House, Inc. Topsham, Maine), Multicycle software (Phoenix Flow Systems, San Diego, Calif. USA) and FACScan (Becton Dickinson, Mountain View, Calif.) software.

A flow cytometer measures the amount of light-emitting substance associated with each cell and other parameters and provides output in the form of, e.g., a histogram, dot plot, or fraction table. The amount of one light-emitting substance associated with each cell can be compared to other properties of that cell, such as the amount of another light-emitting substance to which the cell population has also been exposed, size, granularity, or inherent light-emission.

As sheath fluid containing cells passes through the laser, typically one-by-one, they are exposed to light of various wavelengths. Each particle detected by the cytometer is termed an "event." The degree to which an event transmits or scatters some of the incident light provides a measure of the event's characteristics, e.g., associated light emitting substance. For example, the event may emit light of its own accord or may emit fluorescent light generated by a fluorescent substance introduced into the event. An example of such a substance is a fluorophore-conjugated antibody. The antibody adheres to a component of the event, and the fluorophore responds to the incident light of a particular frequency by emitting light at a known frequency that is detected by, e.g., photomultiplier tubes (PMTs) of the cytometer. The intensity of the emitted or reflected light is measured and stored by the cytometer.

The cytometer compiles emission data into a histogram. The histogram may be reported in one-dimensional form. Alternatively, it may be combined with a histogram of emitted light resulting from other incident wavelengths. Such a combination is typically reported as a "dot plot," in which events are plotted on a grid, and the axes of the grid correspond to the two parameters being measured. For example, events could be exposed to incident light of about 488 nm and assayed for forward light scatter and for emission at the 530 nm wavelength. This wavelength corresponds to a peak emission wavelength for fluorescein isothiocyanate (FITC), a typical fluorochroma used in laser scanning cytometry.

In a typical two-dimensional dot plot output from a laser scanning cytometer, mitotic cells will separate from the nonmitotic G2 population. The mitotic populations may be quantitated relative to one another and to DNA content of the non-dividing population by creating "gates" around them, i.e., by setting threshold values for the minimum and maximum staining levels that will be accepted for establishing DNA content of the mitotic cell.

Similarly, fractions of the cell population will be segregated based on their DNA content. A peak will occur at propidium iodide staining corresponding to the normal 4n DNA content of mitotic cells. Peaks may also occur at higher multiples of the haploid number n, possibly corresponding to polyploid cells. Peaks or above-background plateaus may also occur at propidium iodide staining levels that do not correspond to multiples of haploid number n. These events may correspond to aneuploid cells or cells having an additional portion of a chromosome or lacking a portion of a chromosome. Gates may be formed to distinguish cells falling into various ranges of DNA content from cells with differing DNA content, as described in the Examples.

In two-color flow analysis, the two markers of interest would have fluorescent properties sufficiently different to enable distinguishing them in laser scanning cytometry. For example, the phosphohistone H3 (ser 10) 6G3 monoclonal antibody may be conjugated to FITC. Cells tagged with this antibody would then emit light at approximately 520 nm in response to incident light of approximately 488 nm. In contrast, propidium iodide emits from 560 nm up to 670 nm. PMTs tuned to be sensitive in these ranges may thus distinguish the two signals, from H3-(ser 10) 6G3-FITC and the other from propidium iodide.

Each event may then be gated as either "mitotic" or "non-mitotic" depending on the amount of H3-(ser 10) 6G3 staining. The mitotic cells may then be gated as "euploid," "diploid," "aneuploid," "polyploid," "hyper-diploid," "hypo-diploid" or other, depending on the amount of propidium iodide staining in the non dividing population.

Other methods for identifying mitotic cells and the DNA content of these cells using a mitotic marker and a quantitative DNA stain include histochemistry, immunohistochemistry, immunocytochemistry, and other molecular biology techniques known in the art. These techniques can also be combined with flow cytometric analysis. For example, mitotic cells can be separated out from other cells via flow cytometric. These cells can then be analyzed for DNA content using a non flow cytometric technique.

In one embodiment, the method is used for identifying agents which cause the appearance of numerical chromosomal abnormalities, e.g., polyploidy, in cells. Such agents include cytotoxic agents, e.g., cancer-inducing agents. In one embodiment, the method comprises contacting a cell with a test agent for a time sufficient for the agent to induce the appearance of one or more numerical chromosomal abnormality, if any, in the daughter cells followed by staining the cells with a mitotic marker and a quantitative DNA stain, to determine the amount of DNA at mitosis, such as to detect numerical chromosomal abnormalities, wherein the presence of one or more numerical chromosomal abnormalities in one or more daughter cell indicates that the test agent is an agent that induces numerical chromosomal abnormalities. In another embodiment, the method comprises contacting a cell population with a test agent for a time sufficient for the agent to induce the appearance of one or more numerical chromosomal abnormalities, if any, in a cell followed by staining the cells with a mitotic marker and a quantitative DNA stain, to determine the amount of DNA at mitosis, such as to detect numerical chromosomal abnormalities, wherein the presence of one or more numerical chromosomal abnormalities in one or more cells indicates that the test agent is an agent that induces numerical chromosomal abnormalities. The method of the invention permits the determination of the type of numerical chromosomal abnormality, i.e., whether the abnormality is an aneuploidy or a polyploidy, or an extra or missing portion of chromosomes.

The test agent can be any molecule or complex, such as a small organic molecule. For example, agents that can be tested include potential therapeutics for determining potential aneugenicity. For example, the methods of the invention can be used for testing the aneugenicity of lead pharmaceutical compounds. Compounds found to be aneugenic could then be changed structurally to try to reduce their aneugenicity, each of which compound could be tested for its aneugenicity according to the methods of the invention.

The methods of the invention can also be used for identifying and/or detecting one or more cells with one or more numerical chromosomal abnormality in a population of cells. The population of cells can be, e.g., a biopsy of a subject. The method can be used for determining the presence of abnormal cells, e.g., cancer cells. In fact, it is known that numerous cancerous cells are aneuploid or polyploid. The method of the invention can also be used for determining the stage of a disease which is associated with numerical chromosomal abnormalities, where such stages correlate with the presence of numerical chromosomal abnormalities. Accordingly, the method of the invention can be used to diagnose the presence of a disease in a subject or predict whether a subject is likely to develop a disease.

Exemplary diseases which can be diagnosed with the method of the invention and which have been shown to be associated with chromosomal abnormalities include neoplasms, such as malignant histiocytic disorders such as Malignant Histiocytosis, Acute Monocytic Leukemia, and Large-cell Lymphoma; leukemias such as Hairy Cell Leukemia, T-Cell, HTLV-II-Associated Leukemia, B-Cell Leukemia, T-Cell Leukemia, Acute Lymphocytic Leukemia, Chronic Lymphocytic Leukemia, Mast-Cell Leukemia, Chronic Myeloid Leukemia, Acute Myeloid Leukemia, Philadelphia-Negative Myeloid Leukemia, Philadelphia-Positive Myeloid Leukemia, Acute Myelomonocytic Leukemia, Acute Nonlymphocytic Leukemia, and Plasmacytic Leukemia; lymphatic vessel tumors such as Lymphangioma, Lymphangiomyoma, Lymphangiomyomatosis, and Lymphangiosarcoma; lymphomas such as Hodgkin Disease, Immunoproliferative Small Intestinal Disease, Letterer-Siwe Disease, Non-Hodgkin Lymphoma, B-Cell Lymphoma, Diffuse Lymphoma, Follicular Lymphoma, High-Grade Lymphoma, Intermediate-Grade Lymphoma, Large-Cell Lymphoma, Low-Grade Lymphoma, Mixed-Cell Lymphoma, Small-Cell Lymphoma, T-Cell Lymphoma, Undifferentiated Lymphoma, Plasmacytoma, Multiple Myeloma, Reticuloendotheliosis, and Mast-Cell Sarcoma; mixed and complex tumors such as Adenolymphoma, Pleomorphic Adenoma, Adenomyoma, Adenosarcoma, Adenosquamous Carcinoma, Carcinosarcoma, Hepatoblastoma, Mesenchymoma, Mesodermal Mixed Tumor, Mullerian Mixed Tumor, Myoepithelioma, Nephroblastoma, WAGR Syndrome, Mesoblastic Nephroma, Pulmonary Blastoma, Rhabdoid Tumor, Endometrial Stromal Sarcoma, and Thymoma; connective and soft tissue tumors such as Angiolipoma, Angiomyolipoma, Lipoma, Liposarcoma, Myelolipoma, Chondroblastoma, Chondroma, Chondrosarcoma, Myxoma, Myxosarcoma, Ossifying Fibroma, Giant Cell Tumor of Bone, Osteoblastoma, Osteochondroma, Osteochondromatosis, Osteoma, Osteosarcoma, Ewing's Sarcoma, Dermatofibroma, Fibrous Histiocytoma, Fibroma, Desmoplastic Fibroma, Ossifying Fibroma, Abdominal Fibromatosis, Aggressive Fibromatosis, Fibrosarcoma, Dermatofibrosarcoma, Neurofibrosarcoma, Myofibromatosis, Adenofibroma, Brenner Tumor, Fibroadenoma, Clear Cell Sarcoma, Small Cell Sarcoma, Synovial Sarcoma, Granular Cell Tumor, Leiomyoma, Leiomyosarcoma, Myoma, Rhabdomyoma, Myosarcoma, Rhabdomyosarcoma, Alveolar Soft Part Sarcoma, Hemangiosarcoma, Liposarcoma, Lymphangiosarcoma, Myxosarcoma, Phyllodes Tumor, and Kaposi Sarcoma; germ cell and embryonic tumors such as Embryonal Carcinoma, Chordoma, Dermoid Cyst, Germinoma, Seminoma Mesonephroma, Neuroectodermal Tumors, Teratocarcinoma, Teratoma, and Trophoblastic Neoplasms; glandular and epithelial tumors such as adenomas and adenocarcinomas, Basal Cell Carcinoma, Basal Cell Nevus Syndrome, Basosquamous Carcinoma, Pilomatrixoma, Infiltrating Duct Carcinoma, Noninfiltrating Intraductal Carcinoma, Mammary Paget's Disease, Lobular Carcinoma, Medullary Carcinoma, Pancreatic Ductal Carcinoma, Extramammary Paget's Disease, Intraductal Papilloma, Mucinous Adenocarcinoma, Mucoepidermoid Carcinoma, Signet Ring Cell Carcinoma, Krukenberg Tumor, Cystadenocarcinoma, Cystadenoma, Adenomatoid Tumor, Mesothelioma, Papillary Carcinoma, Squamous Cell Carcinoma, Verrucous Carcinoma, Papilloma, Inverted Papilloma, and Cystic Mesothelioma; neuroepithelial tumors such as Glioma, Astrocytoma, Ependymoma, Ganglioglioma, Gliosarcoma, Medulloblastoma, Oligodendroglioma, Optic Nerve Glioma, Neurocytoma, Pinealoma, and Retinoblastoma; gonadal tissue tumors such as Adrenal Rest Tumor, Androblastoma, Granulosa Cell Tumor, Leydig Cell Tumor, Luteoma, Sertoli Cell Tumor, and Sex Cord-Stromal Tumor; nerve tissue tumors such as Meningioma, Neurilemmoma, Neurofibroma, Plexiform Neurofibroma, Neurofibromatoses, Neurofibrosarcoma, Neuroma, Neurilemmoma, Craniopharyngioma, and Neurothekeoma; vascular tissue tumors such as Angiofibroma, Angiokeratoma, Glomus Tumor, Hemangioma, Hemangioendothelioma, and Hemangiopericytoma; and odontogenic tumors such as Ameloblastoma, Cementoma, Calcifying Odontogenic Cyst, and Odontoma.

Other diseases associated with abnormal ploidy or cellular DNA mass include Klinefelter's syndrome, Turner's syndrome, Down syndrome, Fragile X syndrome, Huntington's syndrome, Triplo-X syndrome, Tetra-X syndrome, Penta-X syndrome, XYY syndrome, monsomies or polysomies of any chromosome including 7, 10, 11, 13, 18, 21, X, and Y, rheumatoid arthritis, and osteoarthritis.

The invention further provides kits for detecting numerical chromosomal abnormalities in cells. The kit may be a kit for identifying compounds which are aneugens or for identifying numerical chromosomal abnormalities in samples. A kit may comprise one marker, such as a mitotic marker or a quantitative DNA stain. In a preferred embodiment, a kit comprises both a mitotic marker and a quantitative DNA stain. Other components that a kit may comprise include additional markers, detection reagents, buffers, fixation reagents, permeabilization reagents, RNase, negative or positive controls, and devices for obtaining a biological sample. The kit may also comprise instructions for use.
The invention also provides compositions comprising a mitotic marker and a quantitative DNA stain.

The present invention is further illustrated by the following examples, which should not be construed as limiting in any way.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2$^{nd}$ Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986).

## Examples

The Examples demonstrate the use of 2 color flow cytometric analysis on cells stained with the mitosis-specific biomarker, phospho-histone H3(ser-10) 6G3 monoclonal antibody (H3-P) for cell cycle analysis and with propidium iodide to assess DNA content. Because the H3-P biomarker distinguishes the mitotic (M) population from the interphase populations (i.e. $G_0G_1$, S and $G_2$), estimates of, e.g., aneuploid and polyploid, cell populations can be easily made by restricting DNA content analysis to mitotic cells. Flow data from 24 hour treatments in purified stimulated human lymphocytes with 3 known aneugens (Noscapine, Colcemid®, and Griseofulvin), a known inducer of polyploidy but not aneuploidy (Cytochasin B) and a toxic compound not expected to induce aneuploidy and/or polyploidy (Potasium Cyanide) are set forth below. The data show that analysis of the mitotic population for numerical chromosomal abnormalities, e.g., aneuploidy and polyploidy, by flow cytometry is comparable to the standard manual microscopic method of analyzing these cell populations. In addition, utilizing the methods described here will dramatically increase the speed of analysis, reduce variability in data and overall improve assessment of numerical aberrations in mitotic populations.

### Example 1: <u>Flow cytometric evaluation of numerical chromosomal abnormalities in Noscapine hydrochloride treated human lymphocyte cultures</u>

Human lymphocytes were obtained from healthy donors by venipuncture in heparinized vacutainer tubes.

Peripheral blood was collected in heparinized vacutainers from healthy male and female volunteers (< 40 years of age). Approximate 10 ml of whole blood was dispensed into a Lymphoprep™ tube (Nycomed Pharma AS Diagnostic, Oslo, Norway) containing ~10 ml of sterile phosphate buffered saline. Following centrifugation for 20 minutes at 2000 rpm, the lymphocyte population was isolated and washed (1X) in PBS. Stock cultures of isolated lymphocytes were established in Williams E medium supplemented with 10% fetal calf serum, 100 U/ml penicillin and 100 ug/ml streptomycin, 2 mM L-glutamine and 1.0% Phytohemmaglutanin-M form (all reagents from Gibco BRL Life Technologies, Inc., Grand Island, NY). The stock cultures were maintained at 37°C in 5% $CO_2$ atmosphere for about 48 hours. The lymphocytes were treated with 0 to 100 µg/ml of the aneugen, Noscapine, (Sigma Chemicals), a known aneugen, for 24 hours at 37°C.

The Noscapine treated and untreated lymphocytes, were then centrifuged for 6 minutes at 1000 rpm, the supernatant was removed and the lymphocytes were resuspended in 5 ml of Phosphate Buffered Saline (PBS; GIBCO BRL). The lymphocytes were then centrifuged, the supernatant removed, and the cells resuspended in 0.5 ml of PBS. The cell suspension was pipetted thoroughly through a 9-inch Pasteur pipette (Kimble Glass Inc.) to ensure single cell suspension. While vortexing a tube containing -20°C (70%) ethanol (about 4.5 ml volume), the cell suspension was added drop by drop. The ethanol solution was prepared by adding 30 ml of distilled water to 70 ml of 200 proof ethyl alcohol from Phamco Products. The cells were then stored at -20°C in the ethanol for at least 24 hours.

The samples and controls for flow analysis were prepared as follows.

(1) <u>H3-P (BLANK):</u>

The cells that were stored at -20°C for at least 24 hours were centrifuged, the supernatant removed, and the cell pellet resuspended in 1-3 ml of 0.1% PBS/Tween 80. This latter solution was prepared by mixing one ml of Tween 80 from Sigma Chemicals to 1000ml of PBS. The cells were incubated for 10-15 minutes at room temperature. The cells were then centrifuged, the supernatant removed and the cell pellet resuspended in 400 μl of primary H3-P antibody solution (Cell Signaling Technology product No. 9706L; the antibody was diluted 1:400 by mixing 36 μl of antibody to 14.4 ml of PBS). The cells were incubated for 40 minutes at 37°C. 5.0 ml of PBS was added to the cells, the cells were centrifuged, the supernatant was removed and the cell pellet was resuspended in 1ml of PBS. The cells were stored as such until analyzed.

(2) H3-P + PI (RED):

The cells that were stored at -20°C for at least 24 hours were stained with the H3-P antibody as described above for the "blank" control. After the 40 minutes incubation with the antibody, 5.0 ml of PBS was added to the cells, the cells were centrifuged, the supernatant was removed and the cell pellet was resuspended in 1ml of propidium iodide/RNase A stain. This latter solution was prepared by mixing 4 mg of RNase A (BD Clontech Product No. 4030-1) and 75 μl of propidium iodide (Molecular Probes product No. P-3566 at 1mg/ml) in 20 ml PBS. The cells were stored on ice until analyzed.

(3) H3-P + Alexa (GREEN):

The cells that were stored at -20°C for at least 24 hours were stained with the H3-P antibody as described above for the "blank" control. After the 40 minutes incubation with the antibody, 5.0 ml of PBS was added to the cells, the cells were centrifuged, the supernatant was removed and the cell pellet was resuspended in 400 μl of Alexa fluor 488 goat antimouse IgG (H+L) conjugate (2 mg/ml). The Alexa fluor reagent was prepared by adding 13.5 μl of Alexa (Molecular Probe product No. A-11011) to 13.5 ml PBS, and storing it away from the light. The cells were incubated for 30 minutes at 37°C. 5.0 ml of PBS was then added to the cells, the cells were centrifuged, the supernatant removed and the cell pellet resuspended in 1.0 ml of PBS. The cells were stored on ice until analyzed.

(4) H3-P + Alexa + PI (DUAL COLOR) for control and treated cultures:

The cells that were stored at -20°C for at least 24 hours were stained with the H3-P antibody and Alexa Fluor as described above for the H3-P + Alexa (GREEN) stained cells. After the incubation of the cells for 30 minutes at 37°C with the Alexa Fluor, 5 ml of PBS was added to the cells, the cells were centrifuged, the supernatant removed and the cell pellet resuspended in 1.0 ml of PI/RNase A stain (as described above). The cells were stored on ice until analyzed.

The cells were analyzed using a Becton Dickinson (BD) Flow Cytometer (488 argon laser), which was calibrated as follows. 1 ml of sheath fluid (FACSFlow, Becton Dickinson Product No. 340398) was added to a capped 12 x 75 mm Falcon 5 ml polystyrene round bottom tube (Product No. 35205). 3 ml of sheath fluid was added to a second capped 12 x 75 mm Falcon 5 ml polystyrene round bottom tube. One drop of unlabeled CaliBRITE™ beads (Becton Dickinson Product No. 349502), was added to each tube after having inverted the unlabeled beads several times prior to dispensing the drop. The first tube was labeled Tube A. One drop each of fluorescein isothiocyanate (FITC) and CaliBRITE™ phycoerythrin (PE) beads was added into the second 5 ml tube (Tube B). Prior to starting the instrument, the sheath reservoir was filed and the waste tank was emptied. Vacuum lines were flushed with sheath fluid, when necessary. The cytometer was turned on and warmed up for about 10 minutes. The computer was turned on and the FACSComp Software (Becton Dickinson Apple Systems 7.5.3 Version 4.0) was opened.

The calibration process was conducted as follows. The CaliBRITE™ Beads lot information was entered in the Set-Up page. The cytometer was set on high speed and switched from standby to run. The 5 ml tube containing water on the cytometer was removed and unlabelled beads (tube A) was placed on the cytometer. "Run" was selected, such that unlabelled beads set the PMTs. When prompted, Tube A was removed and replaced with Tube B. "Run" was selected such that the mixed beads set fluorescence compensation and sensitivity testing. Tube B was then removed and replaced with a 5 ml tube containing water. The cytometer was switched to standby and the calibration report was printed out.

The cytometer was set up for measuring the presence of mitotic cells and DNA analysis as follows. CellQuest™ Software (Becton Dickinson, Version 3.1) was opened. The option "Choose Connect to Cytometer" was chosen from the Acquire menu. The option "Choose Acquisition & Storage" was selected from the Acquire menu. The number of events was set at 10,000 events (default). Then, the "Choose Acquisition & Storage" option was exited. The option "Choose Parameter Description" was selected from the Acquire menu. From the Parameter Description window, a file menu, file storage location, and parameter labels were selected. The option "Choose Counters" from the Acquire menu was selected to view event rate, total events and elapsed time during acquisition. The option "Choose Detectors / Amps" from the Cytometer menu was selected to select the amplifier mode (Lin or Log) for each parameter. The option "Choose Compensation from the Cytometer" menu was chosen to adjust compensation for samples.

Dot plots and histogram plots for acquisition were obtained as follows. "Dot Plots and Histogram Plots" was selected from the tool bar, and were set-up as follows:

1. Acquisition Dot Plot: (X) PARAMETER = FSC-Height 1024 FSC (LIN scale), (Y) PARAMETER = SSC-Height 1024 (LIN scale), No gate, under OPTIONS, SHOW was set to 2000 Dots and then closed.

2. Acquisition Histogram Plot: PARAMETER = FL1-Height 1024 (H3-P), LOG scale, No gate, under OPTIONS, MANUAL SCALE to set to 50, SHOW was set to 2000 events and then closed.

3. Acquisition Histogram Plot: PARAMETER = FL2 Height 1024 (PI), LOG scale, No gate, under OPTIONS, MANUAL SCALE was set to 50, SHOW was set to 2000 events and then closed.

4. Acquisition Histogram Plot: PARAMETER = FL2-Area 1024 (PI), No gate, under OPTIONS, MANUAL SCALE was set to 200, SHOW was set to All events and then closed.

5. Acquisition Dot Plot: (X) PARAMETER = FL2-Area 1024 (PI) LIN scale, (Y) PARAMETER = FL2-Width 1024 (PI) LIN scale, No gate (was set later), under OPTIONS, SHOW was set to All Dots and then closed.

6. Acquisition Dot Plot: (X) PARAMETER = FL2-Area 1024 (PI) LIN scale, (Y) PARAMETER = FL1-Height 1024 (H3-P) LOG scale, No gate (was set later), under OPTIONS, SHOW was set for 5000 Dots and then closed.

Samples were acquired as follows. The cytometer was set to RUN at LOW speed, then the BLANK (no dye) sample was placed on the cytometer. While viewing the FSC/SSC dot plot panel, the Voltage settings were modified for the FSC and SSC parameters in the Detector / Amps window until the BLANK cell sample on the dot plot appeared diagonally across the window at approximately 45° angle. The base of the cell sample was positioned as close to 0 axis- intersect point as possible. The voltage was continued to be adjusted until all or most of the cells appeared in the field. Then the FL1-Height Histogram was viewed, and the voltage of the FL1 detector was increased or decreased until the BLANK cell sample was visible in the left quadrant of the histogram panel. The voltage was adjusted to bring the cell sample as close as possible to the 0 axis-intercept of the plot (the peak all the way to the left of the axis). Then viewing the FL2-Height Histogram, the voltage of the FL2 detector was increased or decreased until the BLANK cell sample was visible in the left quadrant of the histogram panel. The voltage was adjusted to bring the cell sample as close as possible to the 0 axis-intercept of the plot (the peak was all the way to the left of the axis).

The BLANK sample was removed and replaced with the GREEN (Alexa Fluor) sample. While viewing the FL1-Height Histogram, the voltage of the FL1 detector was adjusted to bring the cell sample peak as close to the 0 axis-intercept of the dot plot (the entire cell peak should be visible on the plot). While viewing the FL2-Height Histogram, the Compensation (FL2-% FL1) was adjusted as necessary. There was only one peak in the histogram and that peak was very close to the 0 axis-intercept. In cases in which a small second peak was present, the compensation value was increased until the second peak disappeared.

The GREEN sample was removed and replaced with the RED (PI) sample. While viewing the FL2-Height Histogram, the voltage of the FL2 detector was adjusted to bring the RED cell sample peak over $10^3$ on the (X) axis. This adjustment brought the G1 peak of the RED cell sample over the 200 (X) axis point in the FL2-Area Histogram and the FL2-Area/FL1-Width dot plot. While viewing the FL1-Height Histogram, the Compensation (FL1-% FL2) was adjusted as necessary. There was only one peak in the histogram and that peak was close to the 0 axis-intercept. In cases in which a small second peak was present, the compensation value was increased until the second peak disappeared.

The RED (PI) sample was removed and replaced with DUAL COLOR (Alexa and PI) sample. Final adjustments were made to the voltage to bring the G1 peak of the DUAL COLOR cell sample over the 200 integer of X-axis in the FL2-Area Histogram. This brought the cell sample over the 200 point on the FL2-Area/FL2-Width dot plot. Two to four thousand cells were acquired in set-up mode to identify the cycle population (this population spans from 200 to 400 on the X-axis in untreated cells). A gate was set for measuring DNA distribution by starting slightly to the left of the cycling population then continuing to the far right region of the dot plot ending gate at the 2nd Y axis. The extension of this gate to the 2nd Y-axis of the dot plot was to accommodate cells with greater than 4n DNA. The FL2-Area (PI) / FL1-Height (H3-P) Acquisition Dot Plot was selected and Format Dot Plot was chosen from the tool bar. Gate G1=R2 was selected. This adjustment allowed viewing of the mitotic cells during the acquisition process. The mitotic population appears directly above the G2 population since both contain 4n DNA.

Exited instrument set up mode to acquisition mode. Acquisition for DNA analysis was started and 10,000 ungated cells were acquired. As treated samples were acquired, the movement of the G1 peak on the (X) axis and the extension of the cycling cell population was observed, so that, in cases in which the cycling population of cells fell off the end of the dot plot, the FL2 voltage was later lowered for aneuploidy and polyploidy acquisition (which followed DNA acquisition). The FL2 voltage was optimized as needed per sample (i.e., to keep the G1 peak on 200 of FL2-A x-axis) for Aneuploidy and Polyploidy acquisition. 50,000 ungated cells were acquired from each sample. The data was saved in listmode format.

The presence of aneuploid and polyploid cells in the lymphocytes was determined as follows. CellQuest™ Software was opened, and PLOT and OPEN a dialog box from the tool bar for creating a dot plot. FILE was selected and a data file was opened. PARAMETERS were set up to identify the "cycling cell population": (X) = FL2-Area 1024 (PI) and (Y) = FL2-Width 1024, OPTIONS showed SHOW 100%, No Gate. The dot plot was expanded to the width of the paper. A gate was set for the DNA distribution by starting slightly to the left of the cycling population then continuing to the far right region of the dot plot ending the gate at the 2nd Y axis. The extension of this gate to the 2nd Y-axis of the dot plot was to accommodate cells with greater than 4n DNA (see Fig. 1A). PLOT and OPEN a dialog box was chosen from the tool bar for creating a dot plot. The PARAMETERS were set to identify "cycling cell population": (X) = FL2-Area 1024 (PI) and (Y) = FL1-Height 1024 (H3-P), OPTIONS showed SHOW 100%, and GATE was set at G1=R1.

Using the interphase cells as a guide, the first gate was set over the mitotic cell area above the $G_0G_1$ -S phase areas. Then starting from the end of the first gate, the second gate was set over the mitotic cells above the $G_2$ area (i. e., 4n DNA, which was the largest population of mitotic cells). Starting from the end of the 2nd gate, the 3rd gate was set over the estimated $G_0G_1$-S phase area of the next cell cycle. To estimate this range, the $G_2$ (X) axis value was used as a guide, doubling the value identifies the approximate $G_2$ area of the next cycle, e.g., if $G_2$ is located at 400 on the (X) axis (first cycle), then the second population of cycling $G_2$ cells should appear approximately at 800 on the (X) axis (see Fig. 1). STATS was chosen from the tool bar and REGION GATE was selected. Software automatically calculates % Gated population for each gated region of the dot plots:

Regions (ID):

Gate (1): Region 1 (R1) = Cycling Cell Population

Gate (2): Region 2 (R2) = Hypodiploid Mitotic Population

Gate (3): Region 3 (R3) = Normal Mitotic Cells

Gate (4): Region 4 (R4) = Hyperdiploid Mitotic Population

Gate (5): Region 5 (R5) = Polyploid Mitotic Population

Fig. 2 B is an example of region statistics.

**Regions:**

$R_1$ = Region 1; gated interphase cells
$R_2$ = Region 2; gated mitotic cells with a DNA content (>2n but <4n DNA) (hypodiploid cell population)
$R_3$ = Region 3; gated mitotic cells with a DNA content of 4n (normal cell population)
$R_4$ = Region 4; gated mitotic cells with a DNA content >4n but <8n (hyperdiploid cell population)
$R_5$ = Region 5; gated mitotic cells with a DNA content of 8N (polyploid cell population)

**Calculations**

1. Frequency of hypodiploid cells (%):

$$\%\ \text{hypodiploid cells} = \frac{R_2}{(R_2 + R_3 + R_4 + R_5)} \times 100$$

2. Frequency of hyperdiploid cells (%):

$$\% \text{ hyperdiploid cells} = \frac{R_4}{(R_2 + R_3 + R_4 + R_5)} \times 100$$

3. Frequency of polyploid cells:

$$\% \text{ polyploid cells} = \frac{R_5}{(R_2 + R_3 + R_4 + R_5)} \times 100$$

DNA was analyzed as follows. MODFIT *LT* Software (Verity Software House, Version 3.0Topsham, Maine) was used for this purpose. FILE was chosen from the tool bar and data file was selected. In the Choose Parameter for Analysis dialog box, P6 FL2 A was chosen. In the Choose Gate dialog box, the boxes were left unchecked. MOD was chosen from the tool box. In the Edit Properties for Manual Analysis dialog box the selections were as follows: Autodebris (checked), Autoaggregates (checked), Apoptosis (unchecked), Linearity set at 2.00, Standards (0), Model Template = diploid, Range Position should be set at "Compute Range Position." The RANGE button on the tool bar appeared depressed (activated). The $G_1$ and $G_2$ peaks were automatically identified (see Fig. 3). FIT was chosen on the tool bar and %$G_1$, %$G_2$ and %S phase cells were determined by the MODFIT *LT* software (see Fig. 3).

The results of flow cytometry analysis of human lymphocytes treated with Noscapine, an aneuploidy inducing agent, are shown in Fig. 2. A negative control, i.e., lymphocytes not treated with Noscapine is shown in Fig. 1. The results indicate the presence of hypodiploid cells (in quadrant R2), normal diploid cells (in quadrant R3), hyperdiploid cells (in quadrant R4) and polyploid cells (in quadrant R5) in the population of cells treated with Noscapine. Increases in aberrant cells (i.e., cell observed in R2, R4 and R5) were not observed in the population of cells that was not treated with Noscapine. Hypodiploid cells are cells that have a less DNA than normal cells, whereas hyperdiploid cells are cells which have a more DNA than normal cells.

Thus, this example demonstrates that the flow cytometry analysis of cells stained with a mitotic marker and cells stained with a quantitative DNA stain clearly identifies cells that have an abnormal numerical chromosomal content.

**Example 2: <u>Comparison of flow cytometric and manual analysis of chromosomal abnormality frequencies in cultured human lymphocytes treated with Noscapine</u>**

This Example demonstrates that the cytometric analysis of numerical chromosomal abnormalities described herein gives similar results to a classic manual analysis of numerical chromosomal abnormalities.

Human lymphocytes described above and treated or not with Noscapine as described above were subjected to manual analysis of numerical chromosomal abnormalities for comparison with the number of cells having numerical chromosomal abnormalities (i.e. polyploidy) obtained in Example 1. For manual analysis, samples of cells were centrifuged for 6 minutes at 1000 rpm. The supernatant was removed and the cell pellet was resuspended in 5 ml of 1% pre-warmed (37°C) sodium citrate. The latter solution was prepared by mixing 1 g of citric acid (Sigma Chemicals) in 100 ml of distilled water. The cells were immediately centrifuged as described above. The supernatant was removed and the pellet was vigorously re-suspended in 5 ml of 4°C fixative (3:1 methanol:acetic acid). The cells were then centrifuged as described above and the pellet resuspended in 5 ml of 4°C fixative (3:1 methanol:acetic acid). The cells were then stored overnight at -20°C or until slide preparation.

The slides were prepared as follows. The cells were centrifuged as described above and the supernatant was then poured off. The pellet was re-suspended in about 2 ml of fresh fixative solution. The suspension was dropped on cold wet slides, and the slides were ignited to fix cells to the slide. The slides were stained in 2-10% Giemsa (GIBCO, BRL) solution.

The polyploid index was determined by systematically scoring 1000 consecutive mitotic cells for the frequency of 8n mitotic cells under low magnification (bright field).

The results, which are shown in Fig. 4, indicate that the number of polyploid cells counted at each concentration of Noscapine is similar when counted manually and when counted by flow cytometric evaluation. Thus, the flow cytometric analysis provides data that are essentially identical to those obtained with the classic method of counting polyploid cells.

**Example 3: Flow cytometric analysis of numerical chromosomal abnormalities in human lymphocytes treated with KCN**

Human lymphocytes obtained as described above were incubated in the presence or absence of 0 to 0.04 µg/ml of KCN (FLUKA Chemicals) for 24 hr.... at 37°C temperature. The results, which are shown in Fig. 5, indicate that the percent of aberrant cells (hypodiploid, hyperdiploid or polyploid) is minimal, as can be expected, since KCN is known not to induce numerical aberrations.

**Example 4: Flow cytometric analysis of numerical chromosomal abnormalities in human lymphocytes treated with cytochalasin B**

Human lymphocytes obtained as described above were incubated in the presence or absence of 0 to 6 µg/ml of Cytochalasin B (Sigma Chemicals) for 24 hours at 37°C temperature. The results, which are shown in Fig. 6, indicate that the percent of hyperdiploid cells is minimal, whereas the number of hypodiploid and polyploid cells is high, as can be expected, since Cytochalasin B is known to induce mainly polyploidy.

**Example 5: Flow cytometric analysis of numerical chromosomal abnormalities in human lymphocytes treated with Colcemid®**

Human lymphocytes obtained as described above were incubated in the presence or absence of 0 to 0.04 µg/ml of Colcemid® (GIBCO BRL) for 24 hr.... at 37°C temperature. The results, which are shown in Fig. 7, indicate that the percent of hyperdiploid and hypodiploid cells is increased, and the number of polyploid cells is high, as can be expected, since Colcemid® is known to induce aneuploidy.

**Example 6: Flow cytometric analysis of numerical chromosomal abnormalities in human lymphocytes treated with griseofulvin**

Human lymphocytes obtained as described above were incubated in the presence or absence of 0 to 100 µg/ml of griseofulvin (Sigma Chemicals) for ...24 hours amount of time at ... 37°C temperature. The results, which are shown in Fig. 8, indicate that the percent of hyperdiploid, hypodiploid and polyploid cells is high, as can be expected, since griseofulvin is known to induce aneuploidy.

**Example 7: Flow cytometric analysis of numerical chromosomal abnormalities in human lymphocytes treated with Noscapine**

Human lymphocytes obtained as described above were incubated in the presence or absence of 0 to 100 µg/ml of Noscapine (Sigma Chemicals) for 24 hours amount of time at 37°C temperature. The results, which are shown in Fig. 8, indicate that the percent of hyperdiploid, hypodiploid and polyploid cells is high, as can be expected, since Noscapine is known to induce aneuploidy.

**Equivalents**

Those skilled in the art will recognize, or be able to ascertain using no more than routine experimentation, many equivalents of the specific embodiments of the invention described herein. Such equivalents are intended to be encompassed by the following claims.

**Claims**

1. A method for detecting numerical chromosomal abnormalities, comprising treating a mitotic cell with a mitotic marker and a quantitative DNA stain and quantifying the amount of DNA in the cell using the quantitative DNA stain.

2. A method according to claim 1, wherein the quantifying of DNA comprises the use of flow cytometry.

**3.** A method according to claim 1 or 2, further comprising characterizing the cell as having a numerical chromosomal abnormality, provided the cell is determined by the quantification to contain more DNA or less DNA than that in a cell with normal ploidy.

**4.** A method for identifying a cell having a numerical chromosomal abnormality from a population of cells, comprising treating a population of cells with a mitotic marker and a quantitative DNA stain and identifying a cell from the population as having a numerical chromosomal abnormality, provided the cell is identified as mitotic by the mitotic marker and further provided the cell is determined by the quantitative DNA stain to contain more DNA or less DNA than that in a cell with normal ploidy.

**5.** A method according to claim 4, wherein cell cytometry is used to identify the cell as mitotic and to determine that the cell contains more DNA or less DNA than that in a cell with normal ploidy.

**6.** A method according to any one of claims 1 to 5, wherein the mitotic marker is an agent that detects phosphorylation of a histone that occurs during mitosis.

**7.** A method according to any one of claims 1 to 5, wherein the mitotic marker is a 6G3 monoclonal antibody.

**8.** A method according to any one of claims 1 to 7, wherein the quantitative DNA stain is selected from an intercalating dye, a minor groove binder, a cyanine dye and propidium iodide.

**9.** A method according to any one of claims 1 to 7, wherein the quantitative DNA stain is propidium iodide.

**10.** A method of isolating a cell having a numerical chromosomal abnormality from a population of cells, comprising treating a population of cells with a mitotic marker and a quantitative DNA stain, identifying a cell from the population as having a numerical chromosomal abnormality and isolating the cell, provided the cell is identified as mitotic by the mitotic marker and further provided the cell is determined by the quantitative DNA stain to contain more DNA or less DNA than that in a cell with normal ploidy.

**11.** A method according to claim 10, wherein the cell population contains at least two subpopulations of cells each having a different numerical chromosomal abnormality, and wherein the method further comprises separating the subpopulations of cells from each other and from the cell population.

**12.** An assay for identifying an agent that induces numerical chromosomal abnormalities in a cell, comprising treating a cell with a test agent, treating the cell or its progeny with a mitotic marker and a quantitative DNA stain and quantifying the amount of DNA in the cell using the quantitative DNA stain, provided that the cell or its progeny are mitotic during treatment with a mitotic marker and a quantitative DNA stain.

**13.** An assay according to claim 12, wherein the cell is a peripheral blood mononuclear cell, a lymphocyte, an epithelial cell or a connective tissue cell.

**14.** A kit for detecting numerical chromosomal abnormalities in cells, comprising a mitotic marker and a quantitative DNA stain,

**15.** A composition comprising a mitotic marker and a quantitative DNA stain.

Fig. 1A

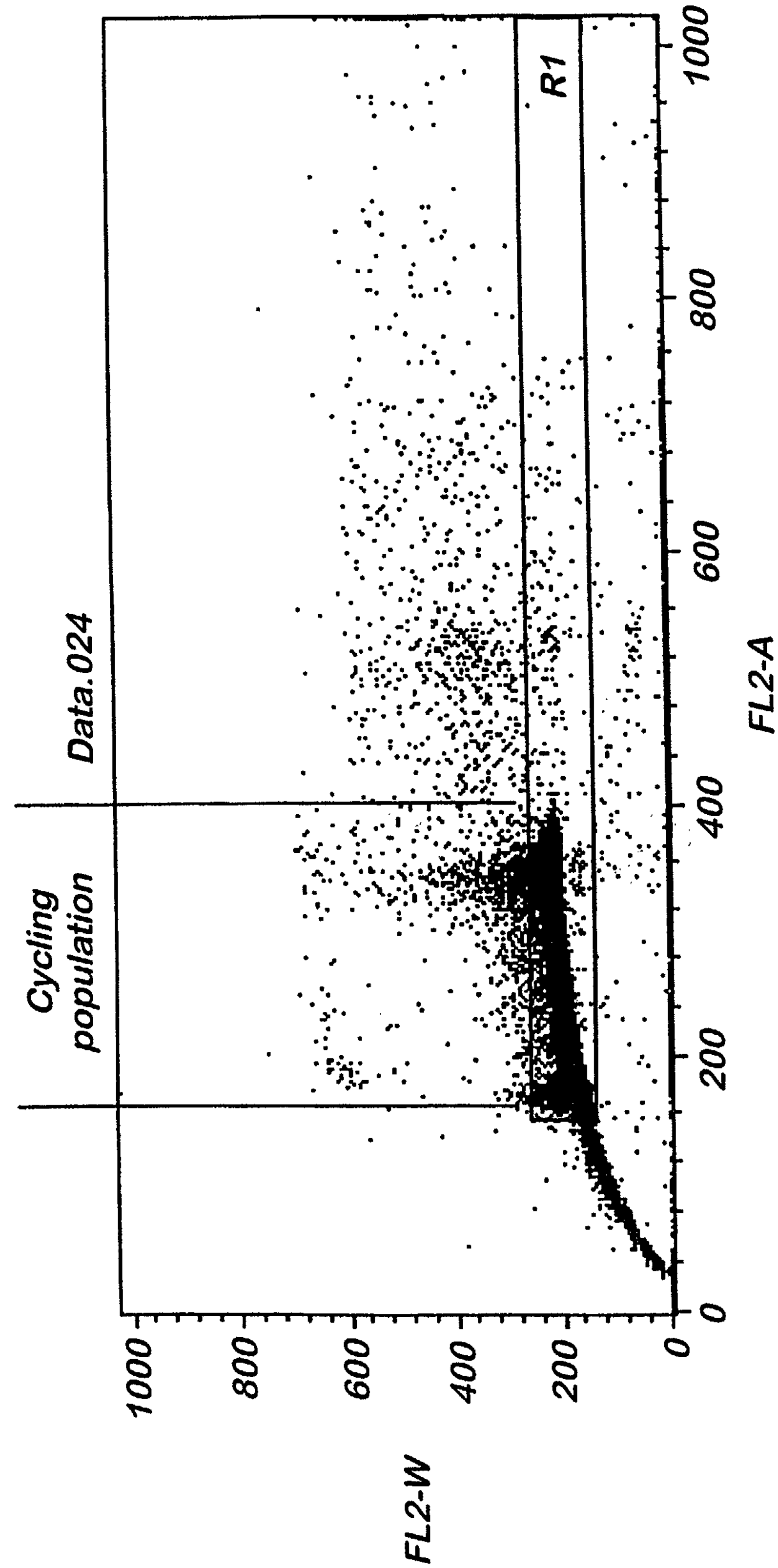

Cycling population
Data.024
R1
FL2-A
FL2-W
0
200
400
600
800
1000

Fig. 1B

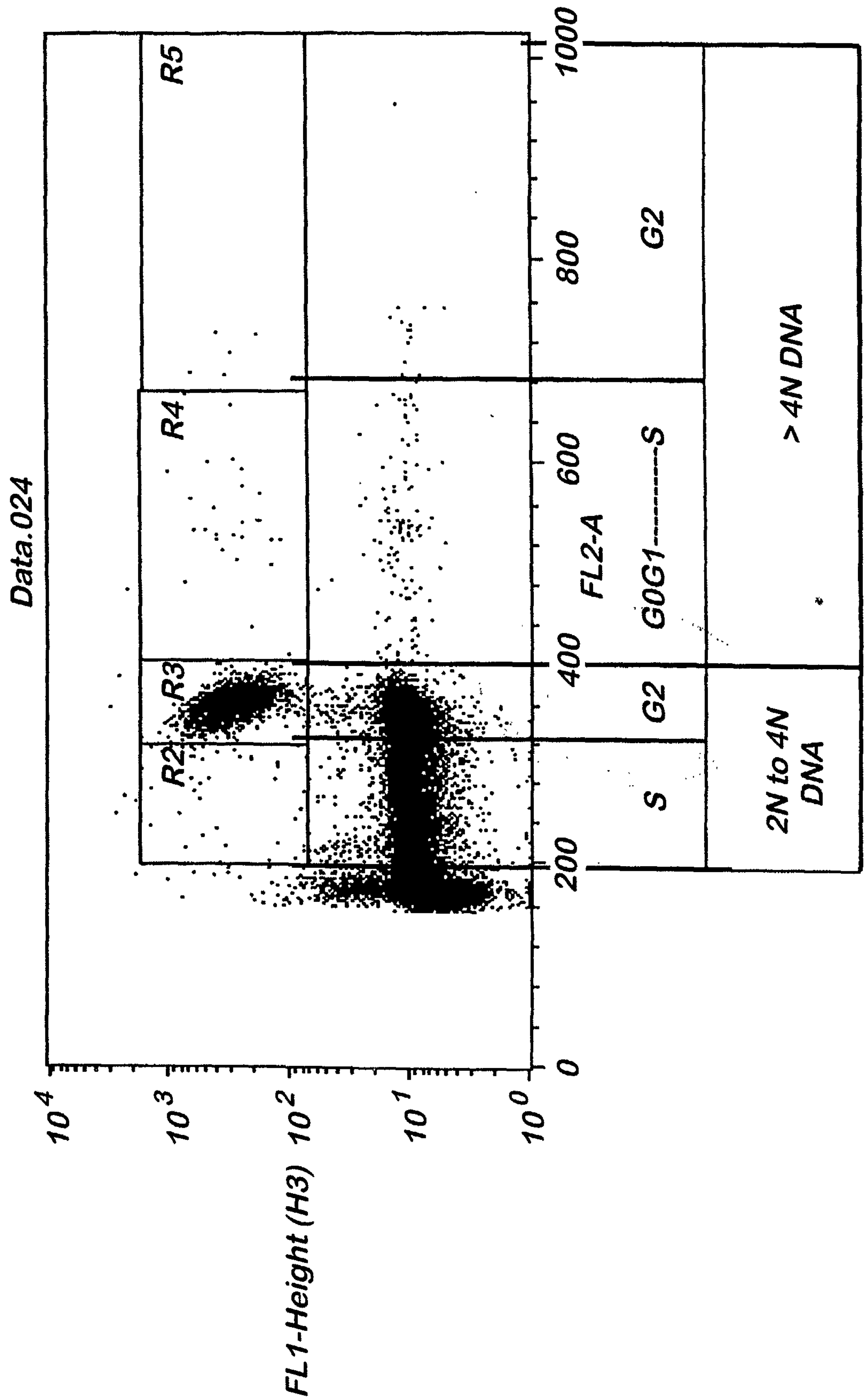
Data.024
FL1-Height (H3)
10^4
10^3
10^2
10^1
10^0
0
200
400
600
800
1000
FL2-A
R2
R3
R4
R5
S
G2
G0G1---------S
G2
2N to 4N DNA
> 4N DNA

Fig. 2A

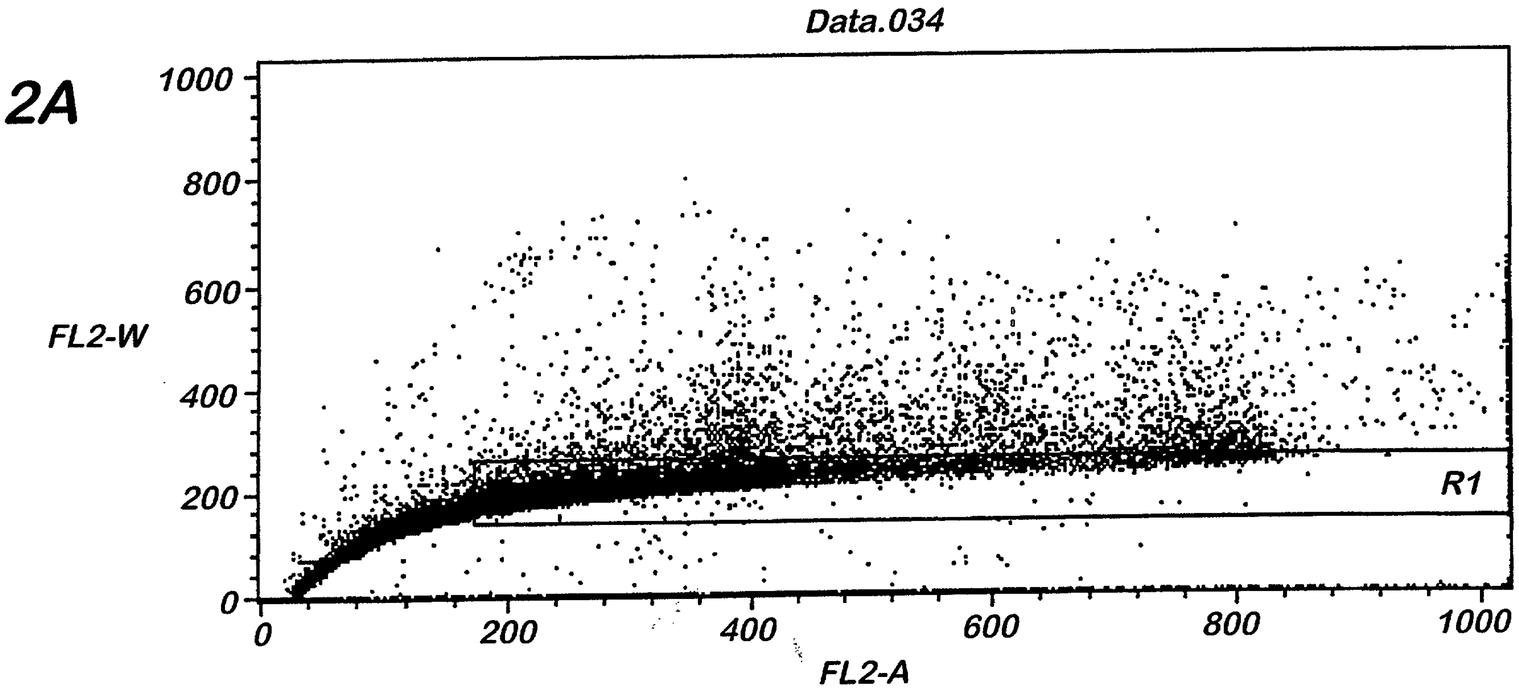


Region Statistics

File: Data.034
Acquisition Date: 02-Nov-01
Gated Events: 24678
X Parameter: FL2-A (Linear)

Log Data Units: Linear Values
Gate: G1
Total Events: 50000
Y Parameter: FL1-H FL1-Height (H3) (Log)

| Region | Events | % Gated | % Total |
|---|---|---|---|
| R1 | 24678 | 100.00 | 49.36 |
| R2 | 117 | 0.47 | 0.23 |
| R3 | 2059 | 8.34 | 4.12 |
| R4 | 215 | 0.87 | 0.43 |
| R5 | 359 | 1.45 | 0.72 |

Fig. 2B

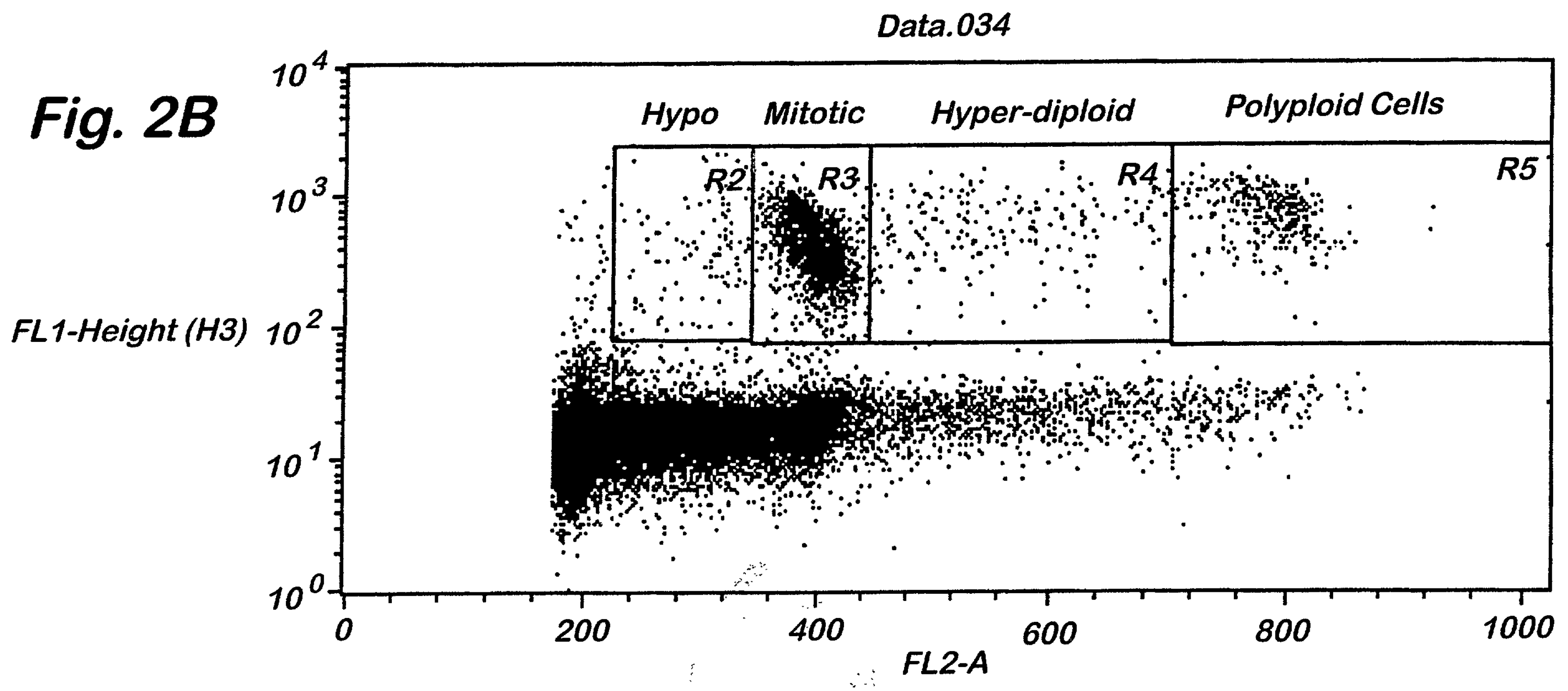


Region Statistics

File: Data.034
Acquisition Date: 02-Nov-01
Gated Events: 24678
X Parameter: FL2-A (Linear)

Log Data Units: Linear Values
Gate: G1
Total Events: 50000
Y Parameter: FL1-H FL1-Height (H3) (Log)

| Region | Events | % Gated | % Total |
|---|---|---|---|
| R1 | 24678 | 100.00 | 49.36 |
| R2 | 117 | 0.47 | 0.23 |
| R3 | 2059 | 8.34 | 4.12 |
| R4 | 215 | 0.87 | 0.43 |
| R5 | 359 | 1.45 | 0.72 |

Fig. 3A

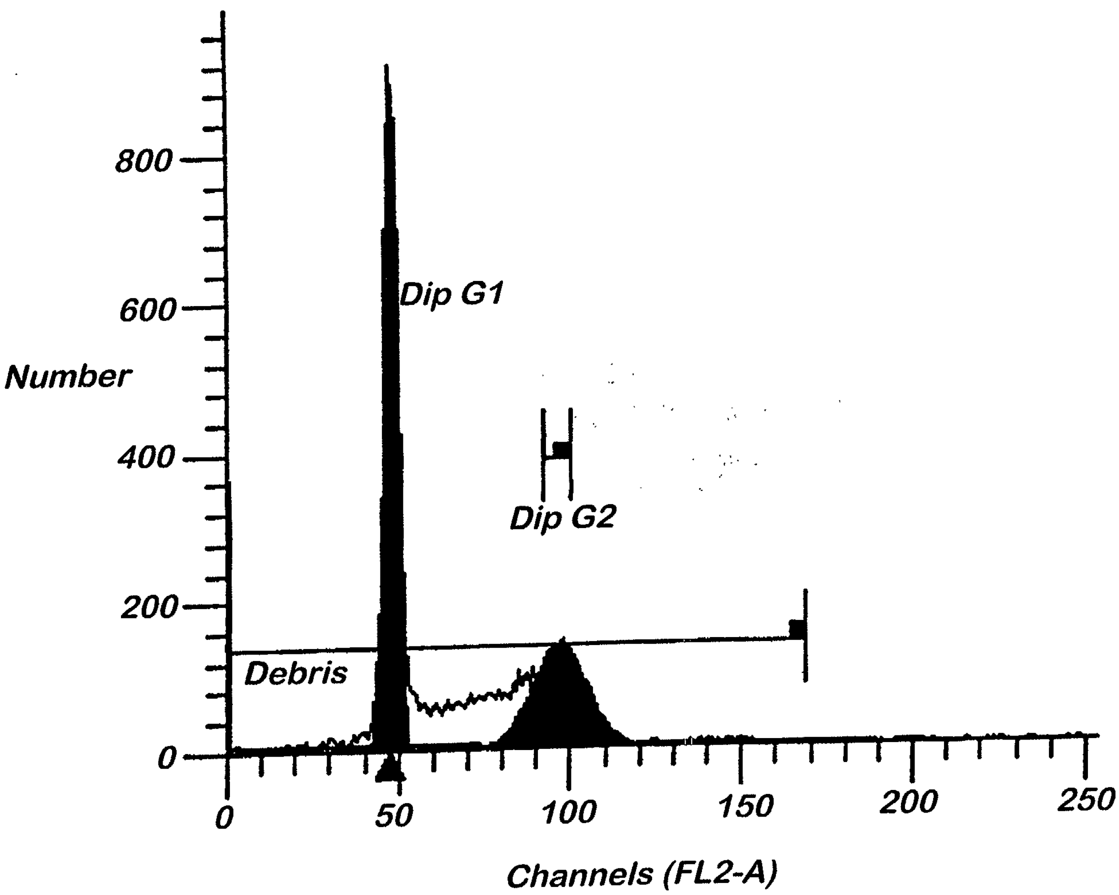
800
600
400
200
0
Number
Dip G1
Dip G2
Debris
0
50
100
150
200
250
Channels (FL2-A)

Fig. 3B

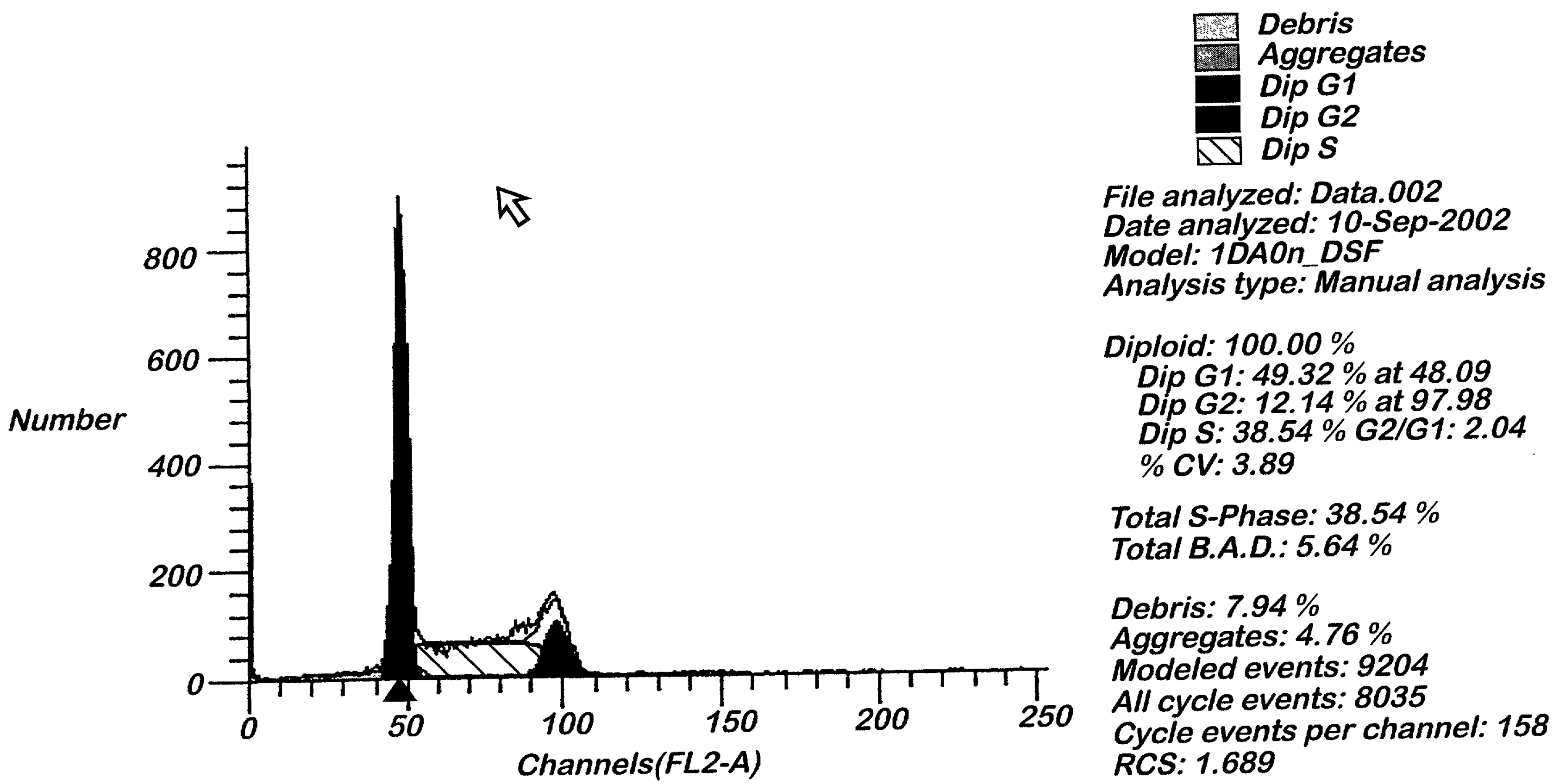
Debris
Aggregates
Dip G1
Dip G2
Dip S
File analyzed: Data.002
Date analyzed: 10-Sep-2002
Model: 1DA0n_DSF
Analysis type: Manual analysis
Diploid: 100.00 %
Dip G1: 49.32 % at 48.09
Dip G2: 12.14 % at 97.98
Dip S: 38.54 % G2/G1: 2.04
% CV: 3.89
Total S-Phase: 38.54 %
Total B.A.D.: 5.64 %
Debris: 7.94 %
Aggregates: 4.76 %
Modeled events: 9204
All cycle events: 8035
Cycle events per channel: 158
RCS: 1.689
Number
800
600
400
200
0
0
50
100
150
200
250
Channels(FL2-A)

Fig. 4

Comparison of Manual and Flow Cytometric Evaluation of Polyploidy in Noscapine Hydrochloride Treated Lymphocytes Cultures

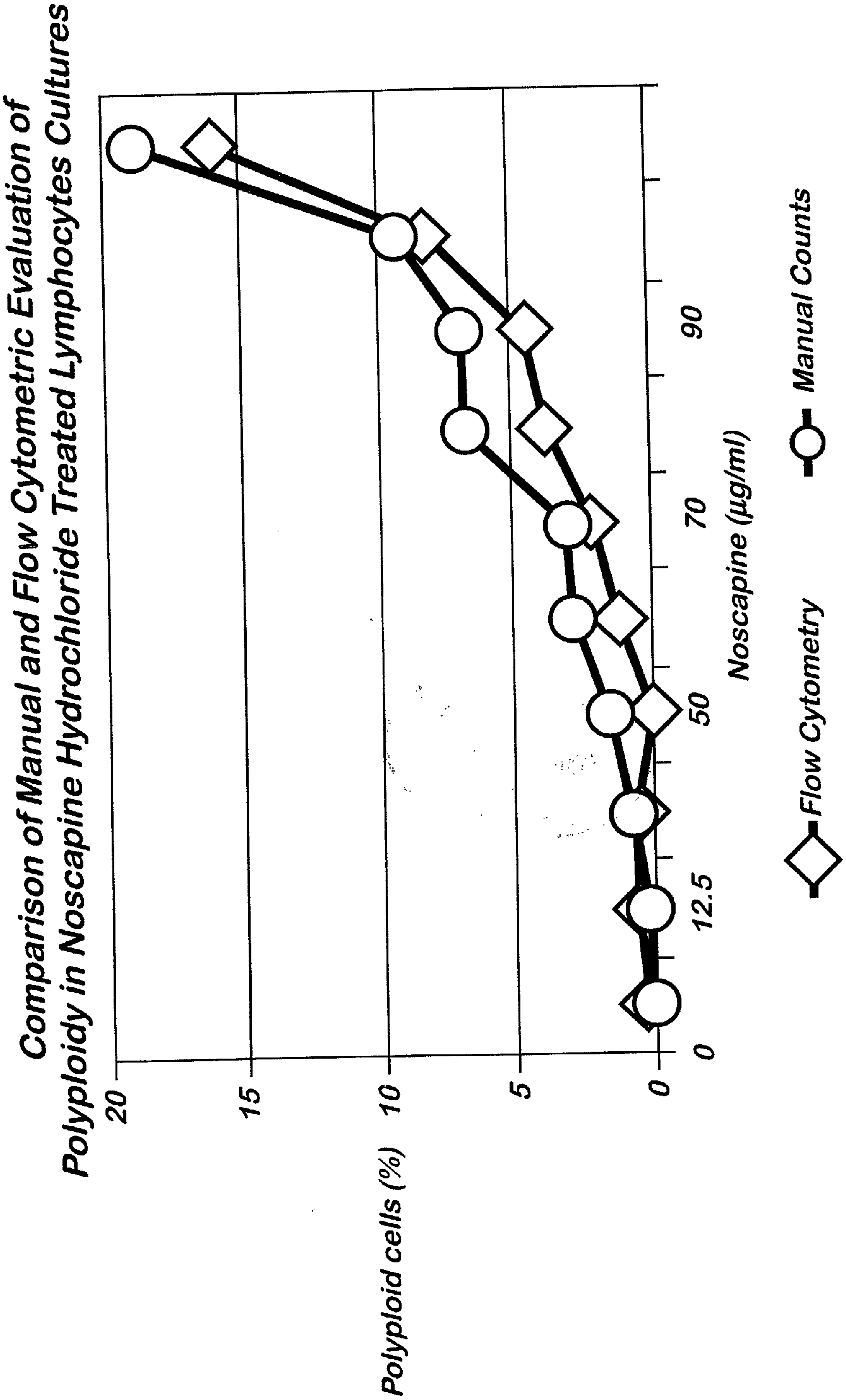

# Fig. 5

**Induction of Hypodiploidy, Hyperdiploidy, and Polyploidy by KCN**

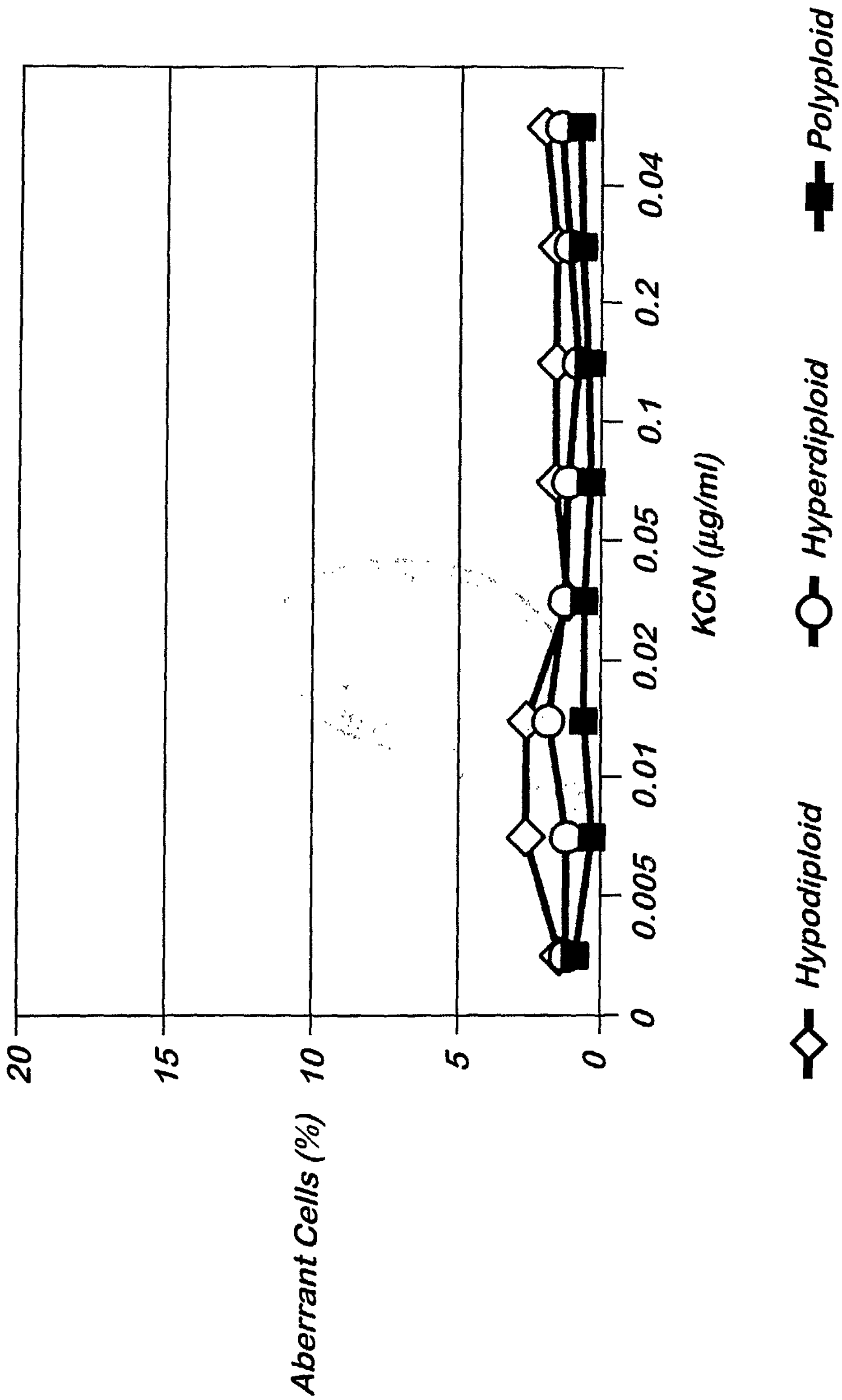

Fig. 6

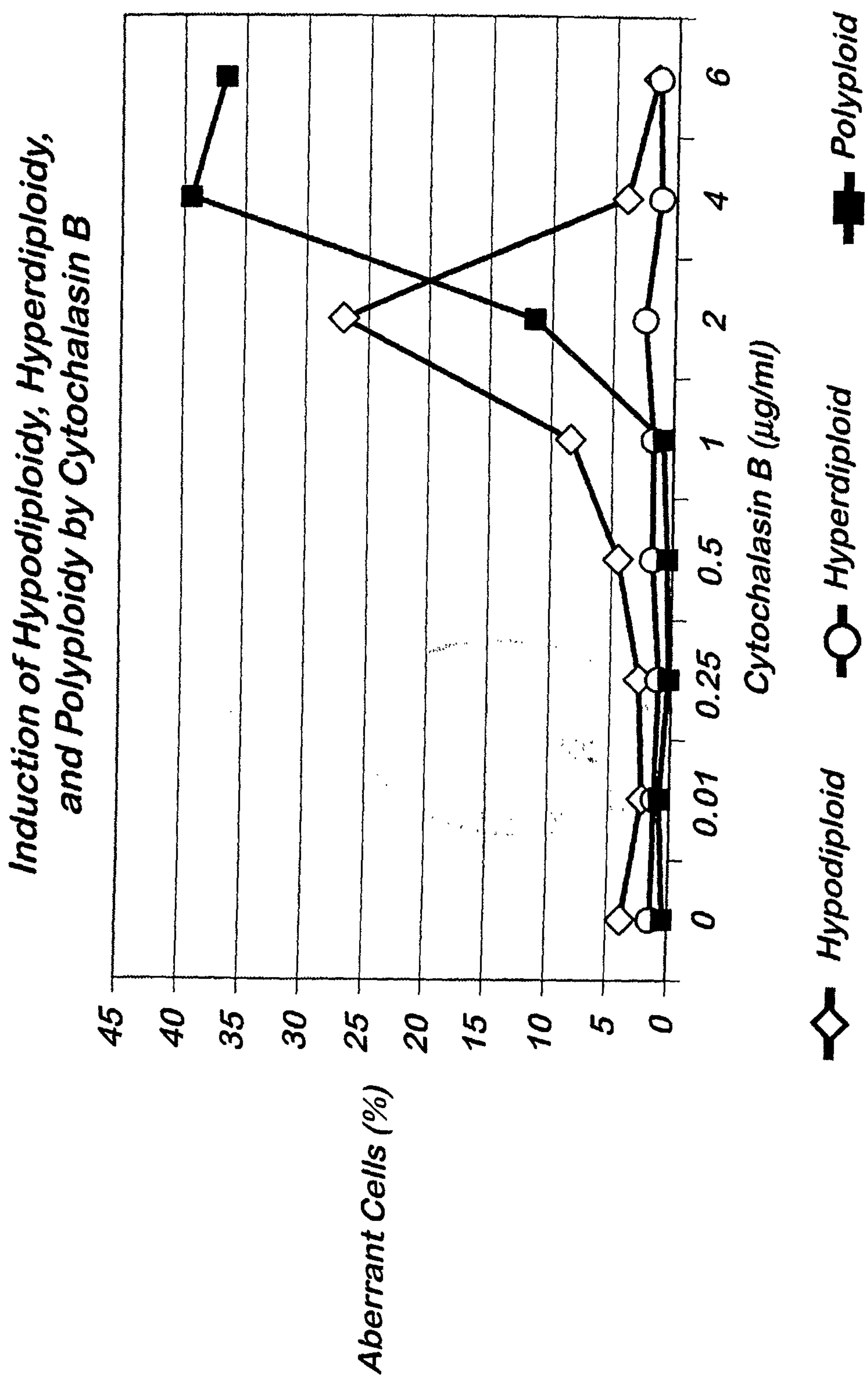
Induction of Hypodiploidy, Hyperdiploidy, and Polyploidy by Cytochalasin B
Aberrant Cells (%)
45
40
35
30
25
20
15
10
5
0
0
0.01
0.25
0.5
1
2
4
6
Cytochalasin B (µg/ml)
Hypodiploid
Hyperdiploid
Polyploid

Fig. 7

Induction of Hypodiploidy, Hyperdiploidy, and Polyploidy by Colcemid

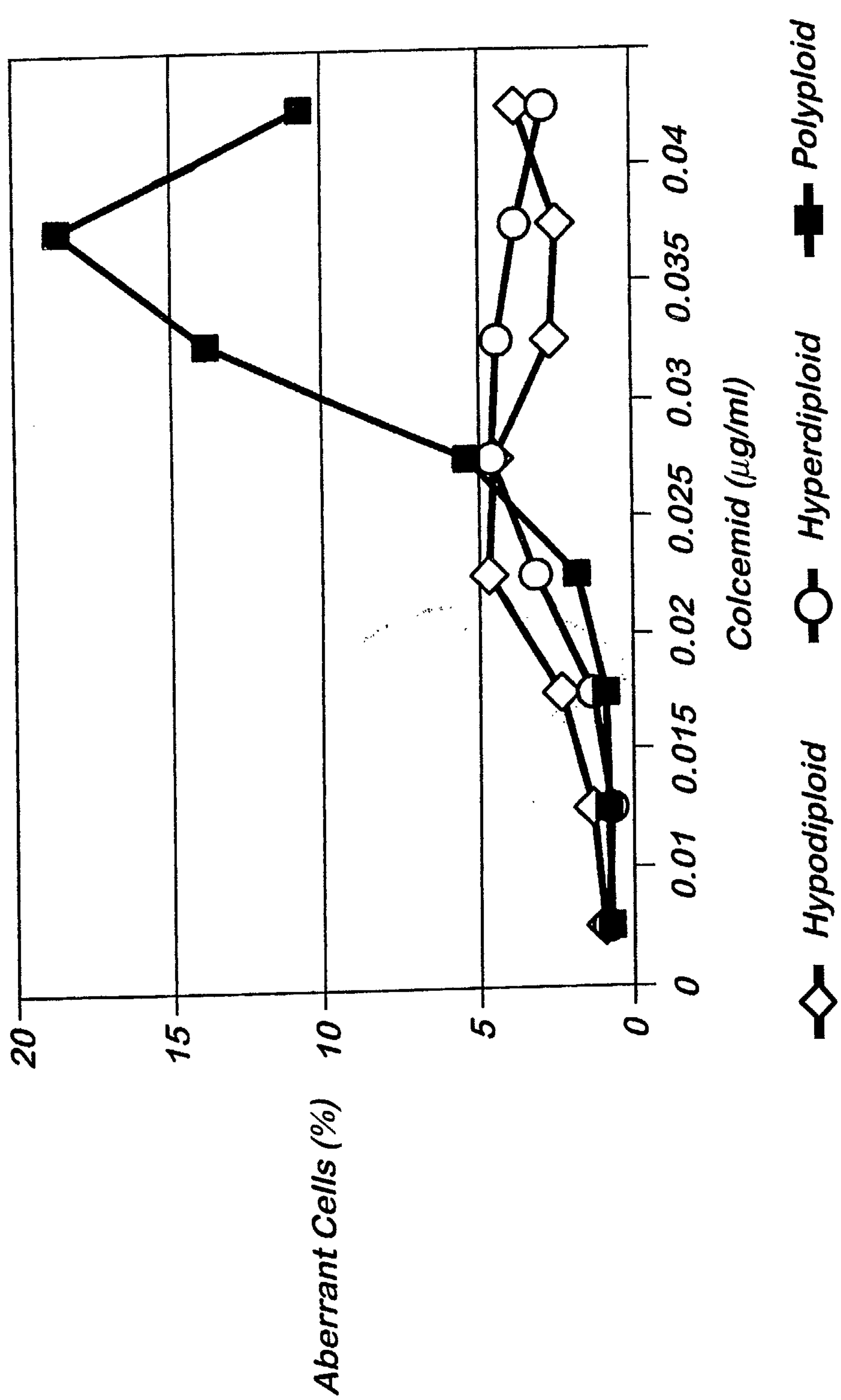

Fig. 8

Induction of Hypodiploidy, Hyperdiploidy, and Polyploidy by Griseofulvin

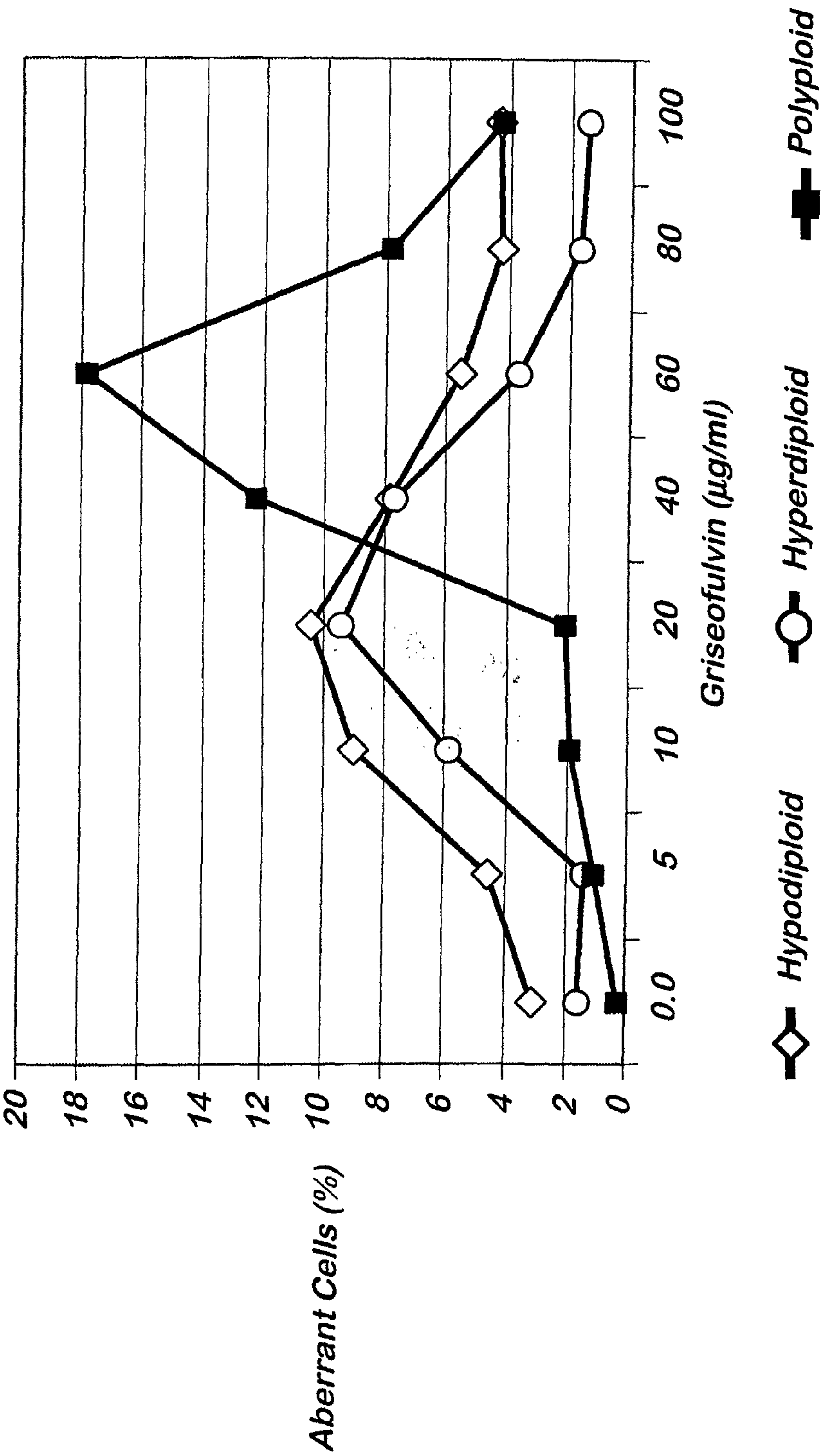

Fig. 9

Induction of Hypodiploidy, Hyperdiploid, and Polyploidy by Noscapine

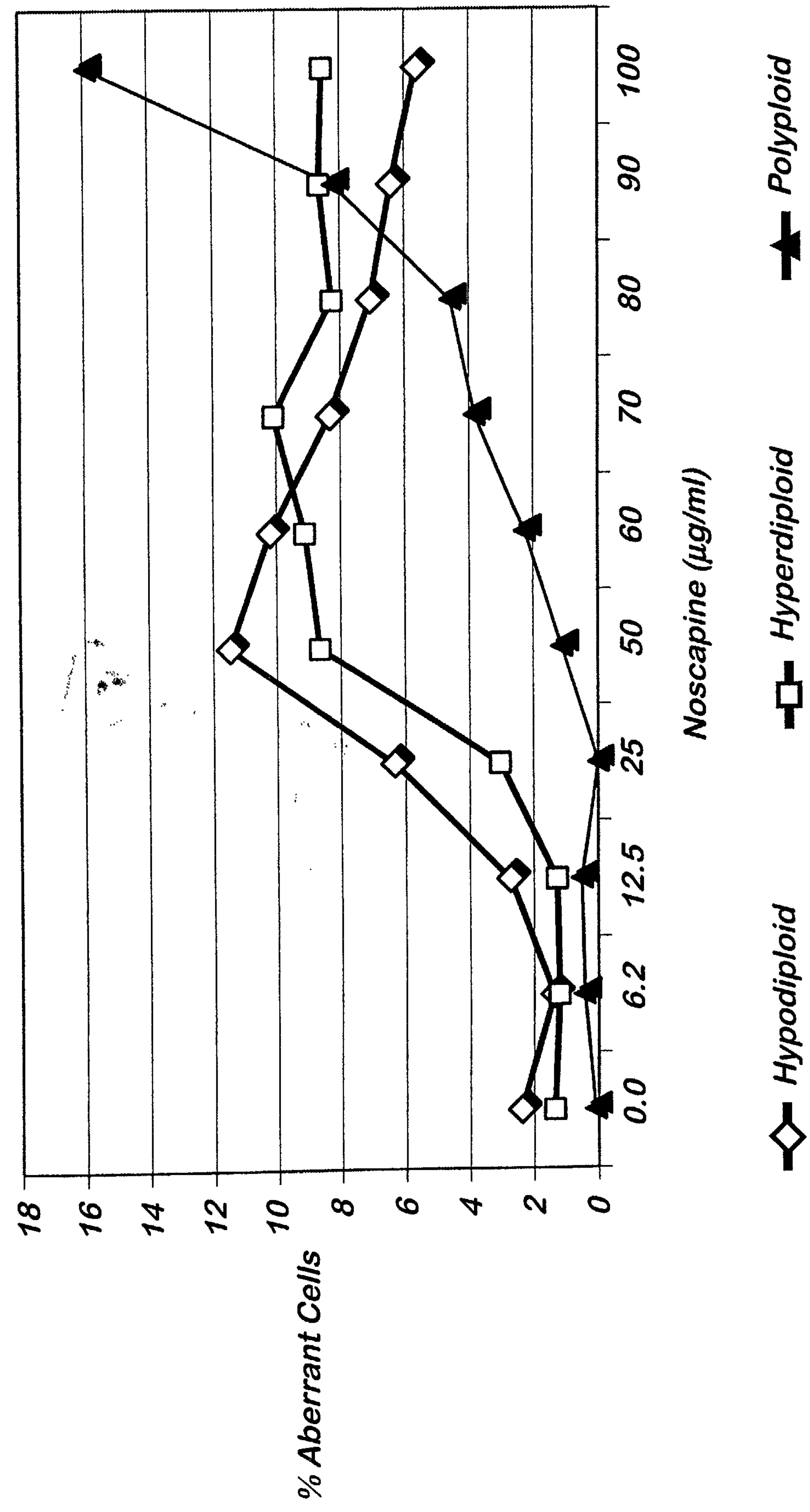

European Patent Office

# EUROPEAN SEARCH REPORT

Application Number
EP 02 25 8072

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 01 04158 A (US HEALTH) 18 January 2001 (2001-01-18) * page 1 - page 4 * * page 12 - page 16 * * page 20 * * page 26 * * claim 15 * | 1-15 | C12Q1/68 |
| Y | US 4 780 406 A (DOLBEARE FRANK A ET AL) 25 October 1988 (1988-10-25) * column 1 - column 5 * * column 8 - column 11 * | 1-15 | |
| Y | EP 0 611 080 A (SINAI SCHOOL MEDICINE) 17 August 1994 (1994-08-17) * column 1 - column 4 * | 1-15 | |
| A | US 6 203 977 B1 (CREMER THOMAS ET AL) 20 March 2001 (2001-03-20) * the whole document * | 1-15 | |
| P,Y | WO 01 92339 A (SCRIPPS RESEARCH INST ;ALLIS C DAVID (US); UNIV VIRGINIA (US); SUL) 6 December 2001 (2001-12-06) * page 1 - page 5 * * claims 12-21 * | 1-15 | |

TECHNICAL FIELDS SEARCHED (Int.Cl.7)
C12Q
G01N

The present search report has been drawn up for all claims

| Place of search | Date of completion of the search | Examiner |
|---|---|---|
| MUNICH | 4 April 2003 | BROCHADO GARGANTA, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 02 25 8072

This annex lists the patent family members relating to the patent documents cited in the above-mentioned European search report.
The members are as contained in the European Patent Office EDP file on
The European Patent Office is in no way liable for these particulars which are merely given for the purpose of information.

04-04-2003

| Patent document cited in search report | Publication date | Patent family member(s) | Publication date |
|---|---|---|---|
| WO 0104158 A | 18-01-2001 | US 6362317 B1 | 26-03-2002 |
| | | AU 5885100 A | 30-01-2001 |
| | | WO 0104158 A1 | 18-01-2001 |
| | | US 2002132989 A1 | 19-09-2002 |
| US 4780406 A | 25-10-1988 | US 4585736 A | 29-04-1986 |
| | | US 4812394 A | 14-03-1989 |
| EP 0611080 A | 17-08-1994 | US 5807999 A | 15-09-1998 |
| | | CA 2113669 A1 | 02-08-1994 |
| | | EP 0611080 A1 | 17-08-1994 |
| | | JP 6245791 A | 06-09-1994 |
| US 6203977 B1 | 20-03-2001 | AT 141957 T | 15-09-1996 |
| | | AU 4642089 A | 12-06-1990 |
| | | CA 2003051 A1 | 15-05-1990 |
| | | CN 1046392 A | 24-10-1990 |
| | | DE 68927059 D1 | 02-10-1996 |
| | | DE 68927059 T2 | 30-01-1997 |
| | | EP 0444115 A1 | 04-09-1991 |
| | | ES 2018959 A6 | 16-05-1991 |
| | | IL 92323 A | 31-10-1995 |
| | | JP 4502855 T | 28-05-1992 |
| | | WO 9005789 A1 | 31-05-1990 |
| | | US 6060251 A | 09-05-2000 |
| | | US 5869237 A | 09-02-1999 |
| WO 0192339 A | 06-12-2001 | AU 7507201 A | 11-12-2001 |
| | | WO 0192339 A1 | 06-12-2001 |

EPO FORM P0459

For more details about this annex : see Official Journal of the European Patent Office, No. 12/82